# EUROPEAN PATENT APPLICATION

(11) **EP 2 695 893 A1**
(43) Date of publication of application: **12.02.2014**
(21) Application number: 12179959.7
(22) Date of filing: 09.08.2012
(51) Int. Cl.: C07K 14/435, C12Q 1/68, G01N 33/58

(54) **Novel far red fluorescent protein**

(71) Applicant: Helmholtz Zentrum München Deutsches Forschungszentrum für Gesundheit und Umwelt GmbH, 85764 Neuherberg (DE)
(72) Inventor: Caldwell, Randolph B., 80939 München (DE); Schötz, Ulrike, 94234 Viechtach (DE); Heuer, Steffen, 85764 Oberschleißheim (DE); Beisker, Wolfgang, 80807 München (DE); Deliolanis, Nikolaos, 70563 Stuttgart (DE)
(74) Representative: Vossius & Partner

(57) **Abstract**

The present invention relates to a nucleic acid molecule encoding a polypeptide having a fluorescence emission activity at a wavelength of at least 630 nm, wherein said nucleic acid molecule is selected from the group consisting of (a) a nucleic acid molecule encoding a polypeptide having the amino acid sequence of SEQ ID NO: 1; (b) a nucleic acid molecule encoding a polypeptide having the amino acid sequence of SEQ ID NO: 1, with the exception that in the polypeptide of SEQ ID NO: 1 (b-i) glutamic acid at the position corresponding to position 16 is replaced by glycine; (b-ii) asparagine at the position corresponding to position 125 is replaced by aspartic acid; and/or (b-iii) lysine at the position corresponding to position 185 is replaced by asparagine; (c) a nucleic acid molecule encoding a polypeptide having at least 94% sequence identity to the polypeptide encoded by the nucleic acid molecule of (a) or (b); (d) a nucleic acid molecule having the nucleotide sequence of SEQ ID NO: 2; (e) a nucleic acid molecule having the nucleotide sequence of SEQ ID NO: 2, with the exception that said nucleic acid molecule has (e-i) at the positions corresponding to positions 48 to 50 of SEQ ID NO: 2 a nucleotide triplet selected from the group consisting of GGT, GGC, GGA and GGG; or (e-ii) at the positions corresponding to positions 375 to 377 of SEQ ID NO: 2 a nucleotide triplet selected from the group consisting of GAT and GAC; and/or (e-iii) at the positions corresponding to positions 555 to 557 of SEQ ID NO: 2 a nucleotide triplet selected from the group consisting of AAT and AAC; (f) a nucleic acid molecule having the nucleotide sequence of (d), (e) or (f), wherein each thymine is replaced by uracile; and (g) a nucleic acid molecule degenerate with respect to the nucleic acid molecule of (d) or (e). The present invention further relates to a vector comprising the nucleic acid molecule of the invention and a non-human host transformed with the vector of the invention. The present invention is further directed to a polypeptide having a fluorescence emission activity at a wavelength of at least 630 nm, a method of producing said polypeptide as well as fusion proteins comprising the polypeptide of the invention. Furthermore, the present invention relates to a diagnostic composition as well as a kit and to methods of detecting the expression of a gene of interest, detecting the activity of a promoter of interest, detecting the presence of a protein of interest and detecting the localization of a polypeptide or a fusion protein of the invention in a cell or tissue.

SEQUENCE LISTING

<110>  Helmholtz Zentrum München

<120>  Novel Far Red Fluorescent Protein

<130>  T2100 EP

<160>  14

<170>  PatentIn version 3.5

<210>  1
<211>  231
<212>  PRT
<213>  Artificial Sequence

<220>
<223>  Amrose v1

<400>  1

Met Ser Glu Leu Ile Lys Glu Asn Met His Met Lys Leu Tyr Met Glu
1               5                   10                  15

Gly Thr Val Asn Asn His His Phe Lys Cys Thr Ser Glu Gly Glu Gly
            20                  25                  30

Lys Pro Tyr Glu Gly Thr Gln Thr Met Lys Ile Lys Val Val Glu Gly
        35                  40                  45

Gly Pro Leu Pro Phe Ala Phe Asp Ile Leu Ala Thr Ser Phe Met Tyr
    50                  55                  60

Gly Ser Lys Ala Phe Ile Asn His Thr Gln Gly Ile Pro Asp Phe Phe
65                  70                  75                  80

Lys Glu Ser Phe Pro Glu Gly Phe Thr Trp Glu Arg Ile Thr Thr Tyr
                85                  90                  95

Glu Asp Gly Gly Val Leu Thr Ala Thr Gln Asp Thr Ser Leu Gln Asn
            100                 105                 110

Gly Cys Leu Ile Tyr Asn Val Lys Ile Asn Gly Val Asn Phe Pro Ser
        115                 120                 125

Asn Gly Pro Val Met Gln Lys Lys Thr Leu Gly Trp Glu Ala Ser Thr
    130                 135                 140

Glu Met Met Tyr Pro Ala Asp Ser Gly Leu Arg Gly His Asn Gln Met
145                 150                 155                 160

Ala Leu Lys Leu Val Gly Gly Gly Tyr Leu His Cys Ser Leu Lys Thr
                165                 170                 175

Thr Tyr Arg Ser Lys Lys Pro Ala Lys Asn Leu Lys Met Pro Gly Phe
            180                 185                 190

His Phe Val Asp Tyr Arg Leu Glu Arg Ile Lys Glu Ala Asp Lys Glu
        195                 200                 205

Thr Tyr Val Glu Gln His Glu Met Ala Val Ala Lys Tyr Cys Asp Leu
    210                 215                 220

Pro Ser Lys Leu Gly His Arg
225                 230

<210>  2
<211>  696
<212>  DNA
<213>  Artificial Sequence

<220>
<223>  Amrose v1

<400>  2
atgagcgagc tgatcaagga gaacatgcac atgaaactgt acatggaggg caccgtgaac      60

aaccaccact tcaagtgcac atccgagggc gaaggcaagc cctacgaggg cacccagacc     120

atgaagatca aggtggtcga gggcggccct ctccccttcg ccttcgacat cctggctacc     180

agcttcatgt acggcagcaa agccttcatc aaccacaccc agggcatccc cgacttcttt     240

aaggagtcct tccctgaggg cttcacatgg gagagaatca ccacatacga agacgggggc     300

gtgctgaccg ctacccagga caccagcctc cagaacggct gcctcatcta caacgtcaag     360

atcaacgggg tgaacttccc atccaacggc cctgtgatgc agaagaaaac actcggctgg     420

gaggccagca ccgagatgat gtaccccgct gacagcggcc tgagaggcca taaccagatg     480

gccctgaagc tcgtgggcgg gggctacctg cactgctccc tcaagaccac atacagatcc     540

aagaaacccg ctaagaacct caagatgccc ggcttccact tcgtggacta cagactggaa     600

agaatcaagg aggccgacaa agagacctac gtcgagcagc acgagatggc tgtggccaag     660

tactgcgacc tccctagcaa actggggcac agatga                               696

<210>  3
<211>  231
<212>  PRT
<213>  Artificial Sequence

<220>
<223>  Amrose v2

<400>  3

Met Ser Glu Leu Ile Lys Glu Asn Met His Met Lys Leu Tyr Met Glu
1               5                   10                  15

Gly Thr Val Asn Asn His His Phe Lys Cys Thr Ser Glu Gly Glu Gly
            20                  25                  30

Lys Pro Tyr Glu Gly Thr Gln Thr Met Lys Ile Lys Val Val Glu Gly
        35                  40                  45

Gly Pro Leu Pro Phe Ala Phe Asp Ile Leu Ala Thr Ser Phe Met Tyr
    50                  55                  60

Gly Ser Lys Ala Phe Ile Asn His Thr Gln Gly Ile Pro Asp Phe Phe
65                  70                  75                  80

Lys Glu Ser Phe Pro Glu Gly Phe Thr Trp Glu Arg Ile Thr Thr Tyr
                85                  90                  95

Glu Asp Gly Gly Val Leu Thr Ala Thr Gln Asp Thr Ser Leu Gln Asn
            100                 105                 110

Gly Cys Leu Ile Tyr Asn Val Lys Ile Asn Gly Val Asp Phe Pro Ser
        115                 120                 125

Asn Gly Pro Val Met Gln Lys Lys Thr Leu Gly Trp Glu Ala Ser Thr
    130                 135                 140

Glu Met Met Tyr Pro Ala Asp Ser Gly Leu Arg Gly His Asn Gln Met
145                 150                 155                 160

Ala Leu Lys Leu Val Gly Gly Gly Tyr Leu His Cys Ser Leu Lys Thr
                165                 170                 175

Thr Tyr Arg Ser Lys Lys Pro Ala Asn Asn Leu Lys Met Pro Gly Phe
            180                 185                 190

His Phe Val Asp Tyr Arg Leu Glu Arg Ile Lys Glu Ala Asp Lys Glu
        195                 200                 205

Thr Tyr Val Glu Gln His Glu Met Ala Val Ala Lys Tyr Cys Asp Leu
    210                 215                 220

Pro Ser Lys Leu Gly His Arg
225                 230

<210>  4
<211>  696
<212>  DNA
<213>  Artificial Sequence

<220>
<223>  Amrose v2

<400>  4

atgagcgagc tgatcaagga gaacatgcac atgaaactgt acatggaggg caccgtgaac 60

aaccaccact tcaagtgcac atccgagggc gaaggcaagc cctacgaggg cacccagacc 120

atgaagatca aggtggtcga gggcggccct ctccccttcg ccttcgacat cctggctacc 180

agcttcatgt acggcagcaa agccttcatc aaccacaccc agggcatccc cgacttcttt 240

aaggagtcct tccctgaggg cttcacatgg gagagaatca ccacatacga agacgggggc 300

gtgctgaccg ctacccagga caccagcctc cagaacggct gcctcatcta caacgtcaag 360

atcaacgggg tggacttccc atccaacggc cctgtgatgc agaagaaaac actcggctgg 420

gaggccagca ccgagatgat gtaccccgct gacagcggcc tgagaggcca taaccagatg 480

gccctgaagc tcgtgggcgg gggctacctg cactgctccc tcaagaccac atacagatcc 540

aagaaacccg ctaacaacct caagatgccc ggcttccact tcgtggacta cagactggaa 600

agaatcaagg aggccgacaa agagacctac gtcgagcagc acgagatggc tgtggccaag 660

tactgcgacc tccctagcaa actggggcac agatga 696

<210> 5
<211> 231
<212> PRT
<213> Artificial Sequence

<220>
<223> Amrose v3

<400> 5

Met Ser Glu Leu Ile Lys Glu Asn Met His Met Lys Leu Tyr Met Gly
1               5                   10                  15

Gly Thr Val Asn Asn His His Phe Lys Cys Thr Ser Glu Gly Glu Gly
            20                  25                  30

Lys Pro Tyr Glu Gly Thr Gln Thr Met Lys Ile Lys Val Val Glu Gly
        35                  40                  45

Gly Pro Leu Pro Phe Ala Phe Asp Ile Leu Ala Thr Ser Phe Met Tyr
    50                  55                  60

Gly Ser Lys Ala Phe Ile Asn His Thr Gln Gly Ile Pro Asp Phe Phe
65                  70                  75                  80

Lys Glu Ser Phe Pro Glu Gly Phe Thr Trp Glu Arg Ile Thr Thr Tyr
                85                  90                  95

Glu Asp Gly Gly Val Leu Thr Ala Thr Gln Asp Thr Ser Leu Gln Asn
            100                 105                 110

Gly Cys Leu Ile Tyr Asn Val Lys Ile Asn Gly Val Asn Phe Pro Ser
        115                 120                 125

Asn Gly Pro Val Met Gln Lys Lys Thr Leu Gly Trp Glu Ala Ser Thr
    130                 135                 140

Glu Met Met Tyr Pro Ala Asp Ser Gly Leu Arg Gly His Asn Gln Met
145                 150                 155                 160

Ala Leu Lys Leu Val Gly Gly Gly Tyr Leu His Cys Ser Leu Lys Thr
                165                 170                 175

Thr Tyr Arg Ser Lys Lys Pro Ala Lys Asn Leu Lys Met Pro Gly Phe
            180                 185                 190

His Phe Val Asp Tyr Arg Leu Glu Arg Ile Lys Glu Ala Asp Lys Glu
        195                 200                 205

Thr Tyr Val Glu Gln His Glu Met Ala Val Ala Lys Tyr Cys Asp Leu
    210                 215                 220

Pro Ser Lys Leu Gly His Arg
225                 230

<210>  6
<211>  696
<212>  DNA
<213>  Artificial Sequence

<220>
<223>  Amrose v3

<400>  6
atgagcgagc tgatcaagga gaacatgcac atgaaactgt acatgggggg caccgtgaac      60

aaccaccact tcaagtgtac atccgagggc gaaggcaagc cctacgaggg cacccagacc     120

atgaagatca aggtggtcga gggcggccct ctccccttcg ccttcgacat cctggctacc     180

agcttcatgt acggcagcaa agccttcatc aaccacaccc agggcatccc cgacttcttt     240

aaggagtcct tccctgaggg cttcacatgg gagagaatca ccacatacga agacgggggc     300

gtgctgaccg ctacccagga caccagcctc cagaacggct gcctcatcta caacgtcaag     360

atcaacgggg tgaacttccc atccaacggc cctgtgatgc agaagaaaac actcggctgg     420

gaggccagca ccgagatgat gtaccccgct gacagcggcc tgagaggcca taaccagatg     480

gccctgaagc tcgtgggcgg gggctacctg cactgctccc tcaagaccac atacagatcc     540

aagaaacccg ctaagaacct caagatgccc ggcttccact tcgtggacta cagactggaa     600

agaatcaagg aggccgacaa agagacctac gtcgagcagc acgagatggc tgtggccaag     660

tactgcgacc tccctagcaa actggggcac agatga                               696

<210>  7

<211>  231
<212>  PRT
<213>  Artificial Sequence

<220>
<223>  Amrose v4

<400>  7

Met Ser Glu Leu Ile Lys Glu Asn Met His Met Lys Leu Tyr Met Gly
1               5                   10                  15

Gly Thr Val Asn Asn His His Phe Lys Cys Thr Ser Glu Gly Glu Gly
            20                  25                  30

Lys Pro Tyr Glu Gly Thr Gln Thr Met Lys Ile Lys Val Val Glu Gly
        35                  40                  45

Gly Pro Leu Pro Phe Ala Phe Asp Ile Leu Ala Thr Ser Phe Met Tyr
    50                  55                  60

Gly Ser Lys Ala Phe Ile Asn His Thr Gln Gly Ile Pro Asp Phe Phe
65                  70                  75                  80

Lys Glu Ser Phe Pro Glu Gly Phe Thr Trp Glu Arg Ile Thr Thr Tyr
                85                  90                  95

Glu Asp Gly Gly Val Leu Thr Ala Thr Gln Asp Thr Ser Leu Gln Asn
            100                 105                 110

Gly Cys Leu Ile Tyr Asn Val Lys Ile Asn Gly Val Asp Phe Pro Ser
        115                 120                 125

Asn Gly Pro Val Met Gln Lys Lys Thr Leu Gly Trp Glu Ala Ser Thr
    130                 135                 140

Glu Met Met Tyr Pro Ala Asp Ser Gly Leu Arg Gly His Asn Gln Met
145                 150                 155                 160

Ala Leu Lys Leu Val Gly Gly Gly Tyr Leu His Cys Ser Leu Lys Thr
                165                 170                 175

Thr Tyr Arg Ser Lys Lys Pro Ala Asn Asn Leu Lys Met Pro Gly Phe
            180                 185                 190

His Phe Val Asp Tyr Arg Leu Glu Arg Ile Lys Glu Ala Asp Lys Glu
        195                 200                 205

Thr Tyr Val Glu Gln His Glu Met Ala Val Ala Lys Tyr Cys Asp Leu
    210                 215                 220

Pro Ser Lys Leu Gly His Arg
225                 230

<210>  8
<211>  696
<212>  DNA
<213>  Artificial Sequence

<220>
<223>  Amrose v4

<400>  8
atgagcgagc tgatcaagga gaacatgcac atgaaactgt acatgggggg caccgtgaac      60

aaccaccact tcaagtgtac atccgagggc gaaggcaagc cctacgaggg cacccagacc     120

atgaagatca aggtggtcga gggcggccct ctccccttcg ccttcgacat cctggctacc     180

agcttcatgt acggcagcaa agccttcatc aaccacaccc agggcatccc cgacttcttt     240

aaggagtcct tccctgaggg cttcacatgg gagagaatca ccacatacga agacgggggc     300

gtgctgaccg ctacccagga caccagcctc cagaacggct gcctcatcta caacgtcaag     360

atcaacgggg tgaacttccc atccaacggc cctgtgatgc agaagaaaac actcggctgg     420

gaggccagca ccgagatgat gtaccccgct gacagcggcc tgagaggcca taaccagatg     480

gccctgaagc tcgtgggcgg gggctacctg cactgctccc tcaagaccac atacagatcc     540

aagaaacccg ctaagaacct caagatgccc ggcttccact tcgtggacta cagactggaa     600

agaatcaagg aggccgacaa agagacctac gtcgagcagc acgagatggc tgtggccaag     660

tactgcgacc tccctagcaa actggggcac agatga                               696

<210>  9
<211>  34
<212>  DNA
<213>  Artificial Sequence

<220>
<223>  primer AI24

<400>  9
cccagatctt gcttgtgaag tcttcttatt gctg                                  34

<210>  10
<211>  40
<212>  DNA
<213>  Artificial Sequence

<220>
<223>  primer AI25

<400>  10
cccgctagcg ccaccatgga cagcctcttg atgaagagga                            40

<210>  11
<211>  32
<212>  DNA

<213>  Artificial Sequence

<220>
<223>  primer rbc580

<400>  11
gtgtgttgga ggtcgctgag tagtgcgcga gc                                    32


<210>  12
<211>  25
<212>  DNA
<213>  Artificial Sequence

<220>
<223>  primer rbc271

<400>  12
ggctgattat gatcctctag agtcg                                            25


<210>  13
<211>  27
<212>  DNA
<213>  Artificial Sequence

<220>
<223>  primer rbc584

<400>  13
agacgggtct gacatggatt ggacgaa                                          27


<210>  14
<211>  36
<212>  DNA
<213>  Artificial Sequence

<220>
<223>  primer rbc276

<400>  14
ccgcccgggt cggcttcggt cggagccatg gagatc                                36

## Description

The present invention relates to a nucleic acid molecule encoding a polypeptide having a fluorescence emission activity at a wavelength of at least 630 nm, wherein said nucleic acid molecule is selected from the group consisting of (a) a nucleic acid molecule encoding a polypeptide having the amino acid sequence of SEQ ID NO: 1; (b) a nucleic acid molecule encoding a polypeptide having the amino acid sequence of SEQ ID NO: 1, with the exception that in the polypeptide of SEQ ID NO: 1 (b-i) glutamic acid at the position corresponding to position 16 is replaced by glycine; (b-ii) asparagine at the position corresponding to position 125 is replaced by aspartic acid; and/or (b-iii) lysine at the position corresponding to position 185 is replaced by asparagine; (c) a nucleic acid molecule encoding a polypeptide having at least 94% sequence identity to the polypeptide encoded by the nucleic acid molecule of (a) or (b); (d) a nucleic acid molecule having the nucleotide sequence of SEQ ID NO: 2; (e) a nucleic acid molecule having the nucleotide sequence of SEQ ID NO: 2, with the exception that said nucleic acid molecule has (e-i) at the positions corresponding to positions 48 to 50 of SEQ ID NO: 2 a nucleotide triplet selected from the group consisting of GGT, GGC, GGA and GGG; or (e-ii) at the positions corresponding to positions 375 to 377 of SEQ ID NO: 2 a nucleotide triplet selected from the group consisting of GAT and GAC; and/or (e-iii) at the positions corresponding to positions 555 to 557 of SEQ ID NO: 2 a nucleotide triplet selected from the group consisting of AAT and AAC; (f) a nucleic acid molecule having the nucleotide sequence of (d), (e) or (f), wherein each thymine is replaced by uracil; and (g) a nucleic acid molecule degenerate with respect to the nucleic acid molecule of (d) or (e). The present invention further relates to a vector comprising the nucleic acid molecule of the invention and a non-human host transformed with the vector of the invention. The present invention is further directed to a polypeptide having a fluorescence emission activity at a wavelength of at least 630 nm, a method of producing said polypeptide as well as fusion proteins comprising the polypeptide of the invention. Furthermore, the present invention relates to a diagnostic composition as well as a kit and to methods of detecting the expression of a gene of interest, detecting the activity of a promoter of interest, detecting the presence of a protein of interest and detecting the localization of a polypeptide or a fusion protein of the invention in a cell or tissue.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

It is generally known in the art (see e.g. the World Wide Web at scholarpedia.org/article/Fluorescent_proteins), that fluorescent proteins are members of a structurally homologous class of proteins that share the unique property of being self-sufficient to form a visible wavelength chromophore. In research, scientists often introduce a gene encoding an engineered fluorescent protein into living cells and subsequently visualize the location and dynamics of the gene product using fluorescence microscopy.

The presence of a fluorescent component in the bioluminescent organs of *Aequorea victoria* jellyfish was first noted by Davenport and Nicol in 1955, but it was only in 1962 that it was realized that this fluorophore is a protein (Shimomura et al. 1962). The protein sequence derived from the cDNA nucleotide sequence (Prasher et al., 1992) contains 238 amino acid residues and led to the determination of the chromophore structure, consisting of the residues Ser65, Tyr66 and Gly67. The first demonstrations that the gene was self-sufficient to undergo the post-translational modifications necessary for chromophore formation was reported by Chalfie *et al.* in 1994, showing that the gene encoding *Aequorea* green fluorescent protein (GFP) could be functionally expressed in the sensory neurons of the nematode *Caenorhabditis elegans* (Chalfie et al. 1994). Also Inouye and Tsuji showed that expression of the gene in *Escherichia coli* resulted in green fluorescent bacteria (Inouye and Tsuji 1994).

Lately, additional homologous fluorescent proteins from distantly related sea creatures have been discovered. The fluorescent properties of such fluorescent proteins make them an especially useful tool in living cells and tissue. Substantial trials were carried out over the years to optimize the properties of wild-type GFP. The earliest modifications of wild-type GFP were directed at improving the ability to express the protein in mammalian cell systems at 37°C after it was previously shown to express in prokaryotic cells. Other improvements included simplification of the extinction spectrum, increasing the fluorescence intensity of the protein by increasing the extinction coefficient as well as increasing the speed and efficiency of folding, reducing the sensitivity to pH and halides and more recently reducing the tendency of the protein to dimerize. Another limitation of *Aequorea* green fluorescent protein is that it was originally available in just the one fluorescent color. This limitation was overcome through the generation of a series of variants with distinct excitation and emission maxima, such as e.g. blue fluorescent protein (BFP), cyan fluorescent protein (CFP), yellow fluorescent protein (YFP), a violet-excitable green fluorescent variant known as Sapphire and a cyan-excitable green fluorescing variant known as enhanced green fluorescent protein (EGFP), which has the S65T and F64L mutations responsible for spectral shift, stability, brightness and folding. In addition, red shifted GFPs were developed, such as e.g. R10-3 or GFP red shifted (rsGFP), which, however are not truly red, and a Discosoma species red fluorescent protein, termed DsRed.

While such enhanced green fluorescent proteins with increased brightness are desirable, the more sought after fluorescent proteins for intravital and whole-body imaging are those emitting nearer to and into the infrared (IR) spectrum (Deliolanis *et al.,* 2008 and Shcherbo *et al.,* 2007), e.g. far-red fluorescent proteins. Shcherbo *et al.,* 2007 for example describe a far-red fluorescent protein named Katushka, which is seven- to tenfold brighter as compared to previously described far-red fluorescent proteins such as HcRed or mPlum. Katuschka as well as its monomeric variant mKate have excitation and emission spectra of 588nm and 635nm, respectively. A further development of mKate was described by the same authors in Shcherbo *et al.* 2009. mKate2 is almost 3-fold brighter than mKate and provides improved pH-stability, thus increasing brightness at physiological pH. The excitation and emission spectra of 588 nm and 635nm, respectively, remain identical to those found for mKate.

For optimum whole-body usability, the spectrum of a far-red fluorescent protein should reside in the window where tissue penetration by light is at a maximum and interference by hemoglobin, tissue chromophores and water are at a minimum. The optical window where there is less light absorption and scattering lies between 600nm and 1200nm (Parrish, 1981). More precisely, the absorption coefficient of tissue drops rapidly in the 590nm to 630nm window making fluorescent proteins with strong absorbance at above 630nm particularly interesting. Further attenuating the window of opportunity is the variation in tissue densities effectively giving an upper limit in the 930nm to 1000nm range, whereby absorption by lipid and water become interfering factors (Shcherbo *et al.,* 2009).

Despite the fact that a lot of effort has been invested into improved far-red fluorescent proteins, there still remain problems in advanced whole-body imaging methods, such as optical fluorescent tomography and multispectral opto-acoustic tomography, for which the effective diagnostic and therapeutic window is in the 630nm to 900nm range (Shu *et al.,* 2009). While 633 nm red lasers are widely used in research, many of the commercially available red fluorescent proteins are not sufficiently excitable at this wavelength. Furthermore, multi-color labeling requires fluorescent proteins with no or only minimal residual fluorescence in the green, yellow or blue spectrum in order to allow for the simultaneous imaging with fluorescent proteins of these spectra without high levels of background signal. Thus, there is still a need to provide improved far-red fluorescent proteins.

This need is addressed by the provision of the embodiments characterized in the claims.

Accordingly, the present invention relates to a nucleic acid molecule encoding a polypeptide having a fluorescence emission activity at a wavelength of at least 630 nm, wherein said nucleic acid molecule is selected from the group consisting of (a) a nucleic acid molecule encoding a polypeptide having the amino acid sequence of SEQ ID NO: 1; (b) a nucleic acid molecule encoding a polypeptide having the amino acid sequence of SEQ ID NO: 1, with the exception that in the polypeptide of SEQ ID NO: 1 (b-i) glutamic acid at the position corresponding to position 16 is replaced by glycine; (b-ii) asparagine at the position corresponding to position 125 is replaced by aspartic acid; and/or (b-iii) lysine at the position corresponding to position 185 is replaced by asparagine; (c) a nucleic acid molecule encoding a polypeptide having at least 94% sequence identity to the polypeptide encoded by the nucleic acid molecule of (a) or (b); (d) a nucleic acid molecule having the nucleotide sequence of SEQ ID NO: 2; (e) a nucleic acid molecule having the nucleotide sequence of SEQ ID NO: 2, with the exception that said nucleic acid molecule has (e-i) at the positions corresponding to positions 48 to 50 of SEQ ID NO: 2 a nucleotide triplet selected from the group consisting of GGT, GGC, GGA and GGG; or (e-ii) at the positions corresponding to positions 375 to 377 of SEQ ID NO: 2 a nucleotide triplet selected from the group consisting of GAT and GAC; and/or (e-iii) at the positions corresponding to positions 555 to 557 of SEQ ID NO: 2 a nucleotide triplet selected from the group consisting of AAT and AAC; (f) a nucleic acid molecule having the nucleotide sequence of (d), (e) or (f), wherein each thymine is replaced by uracile; and (g) a nucleic acid molecule degenerate with respect to the nucleic acid molecule of (d) or (e).

In accordance with the present invention, the term "nucleic acid molecule" includes DNA, such as cDNA or genomic DNA, and RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8:1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art. The term "nucleic acid molecule" is used interchangeably with the term "polynucleotide" herein. Preferably, the nucleic acid molecule is DNA.

The term "polypeptide", in accordance with the present invention, describes linear molecular chains of amino acids, including single chain polypeptides or their fragments, containing more than 30 amino acids. The term "fragment" in accordance with the present invention refers to a portion of the polypeptide comprising at least the amino acid residues necessary to maintain the biological activity of the polypeptide. Polypeptides may further form oligomers consisting of at least two identical or different molecules. The corresponding higher order structures of such multimers are, correspondingly, termed homo- or heterodimers, homo- or heterotrimers etc.. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" are used interchangeably herein and also refer to naturally modified polypeptides wherein the modification is effected e.g. by glycosylation, acetylation, phosphorylation and the like. Such modifications are well known in the art.

The term "having the sequence", as used herein, encompasses polypeptides or nucleic acid molecules comprising the recited sequence as well as polypeptides or nucleic acid molecules consisting of the recited sequence.

Fluorescence is the result of a three-stage process that occurs in certain molecules called fluorophores or fluorescent dyes, *inter alia* fluorescent proteins. This is conveniently illustrated by an electronic-state diagram (Jablonski diagram) easily retrievable by a person skilled in the art. A fluorescent probe is a fluorophore designed to localize within a specific region of a biological specimen or to respond to a specific stimulus.
In a first stage, a photon of energy hv_{EX} is supplied by an external source such as an incandescent lamp or a laser and absorbed by the fluorophore, creating an excited electronic singlet state (S₁'). This process distinguishes fluorescence from chemiluminescense, in which the excited state is populated by a chemical reaction. The ability of a fluorescent protein to absorb this energy and reach an excited state is also referred to herein as "absorption/excitation activity".
The second, excited state exists for a finite time, typically 1 to 10 nanoseconds. During this time, the fluorophore undergoes conformational changes and is also subject to a multitude of possible interactions with its molecular environment. These processes have two important consequences. First, the energy of S₁' is partially dissipated, yielding a relaxed singlet excited state (S₁) from which fluorescence emission originates. Second, not all the molecules initially excited by absorption (first stage) return to the ground state (S₀) by fluorescence emission. Other processes such as collisional quenching, fluorescence resonance energy transfer (FRET) and intersystem crossing may also depopulate S₁. The fluorescence quantum yield, which is the ratio of the number of fluorescence photons emitted (stage 3) to the number of photons absorbed (first stage), is a measure of the relative extent to which these processes occur.
In the third stage, a photon of energy hv_{EM} is emitted, returning the fluorophore to its ground state S₀. The ability of a fluorescent protein to emit this energy is also referred to herein as "emission activity". Due to energy dissipation during the excited-state lifetime, the energy of this photon is lower, and therefore of longer wavelength, than the excitation photon hv_{EX}. The difference in wavelengths between the peak excitation and emission of a fluorescent species represented by (hv_{EX} - hv_{EM}) is called the Stokes shift. The Stokes shift is fundamental to the sensitivity of fluorescence techniques because it allows emission photons to be detected against a low background, isolated from excitation photons.

The entire fluorescence process is cyclical. Unless the fluorophore is irreversibly destroyed in the excited state (an important phenomenon known as photo-bleaching), the same fluorophore can be excited and detected repeatedly. The fact that a single fluorophore can generate many thousands of detectable photons is fundamental to the high sensitivity of fluorescence detection techniques. For polyatomic molecules in solution, the discrete electronic transitions represented by hv_{EX} and hv_{EM} are replaced by rather broad energy spectra called the fluorescence excitation spectrum and fluorescence emission spectrum, respectively. The bandwidths of these spectra are parameters of particular importance for applications in which two or more different fluorophores are simultaneously detected. With few exceptions, the fluorescence excitation spectrum of a single fluorophore species in dilute solution is identical to its absorption spectrum. Under the same conditions, the fluorescence emission spectrum is independent of the excitation wavelength, due to the partial dissipation of excitation energy during the excited-state lifetime. The emission intensity is proportional to the amplitude of the fluorescence excitation spectrum at the excitation wavelength.

The term "fluorescence emission activity at a wavelength of at least 630nm", in accordance with the present invention, relates to the requirement that the polypeptide encoded by the nucleic acid molecule of the invention is capable of emitting the energy hv_{EM} at a wavelength of 630nm or higher after being excited by a stimulus that is supplied by an external source. More preferable, the polypeptide has a fluorescence emission activity at a wavelength of at least 645 nm, more preferably at least 670 nm, even more preferably at least 690nm, such as e.g. at least 750nm, such as 790nm and most preferably at least 850 nm.
It will be appreciated that the term "fluorescence emission activity" relates to the ability of emission at the recited wavelength and does not necessitate that the emission peak of said polypeptide is at this wavelength. Accordingly, encompassed is any polypeptide having an emission activity at the recited wavelength of at least 90% of the emission activity observed at the peak, such as e.g. at least 80%, at least 70%, at least 60%, at least 50%, at least 40%, at least 30%, at least 20%, at least 10% or even at least 1% of the emission activity observed at the peak. Means and methods to determine whether a polypeptide has the required activity are well known in the art and have been described, for example, in Joseph R. Lakowicz and Barry R. Masters, "Principles of Fluorescence Spectroscopy, Third Edition", J. Biomed. Opt. 13, 029901 (Apr 30, 2008); doi:10.1117/1.2904580; and are also shown in the examples below.

The polypeptide having the amino acid sequence of SEQ ID NO: 1 is also referred to herein as Amrose v1 and comprises in position 82 glutamic acid (E), in position 147 methionine (M), in position 158 asparagine (N) and in position 197 tyrosine (Y). In these four positions, Amrose v1 differs from eqFP615 (from Evrogen JSC), from which it was derived. In other words, compared to eqFP615 the following mutations are present in Amrose v1 (SEQ ID NO:1) Q82E, L147M, S158N and H197Y. The polypeptide having the amino acid sequence of SEQ ID NO: 1 is for example encoded by the nucleic acid molecule having the sequence of SEQ ID NO: 2.

The term "at the position corresponding to position [...]", in relation to SEQ ID NO:1, refers to the recited position shown in the sequence according to SEQ ID NO:1, e.g. the position corresponding to position 16 is glutamic acid in SEQ ID NO:1, the position corresponding to position 125 is asparagine in SEQ ID NO:1 and the position corresponding to position 185 is lysine in SEQ ID NO:1.

In accordance with the present invention, the nucleic acid molecule encoding a polypeptide having a fluorescence emission activity at a wavelength of at least 630 nm can be a nucleic acid molecule that encodes a polypeptide having additional mutations as compared to the polypeptide according to SEQ ID NO:1. These mutations include the substitution of glutamic acid with glycine (E16G) at the position corresponding to position 16 in SEQ ID NO: 1, the substitution of asparagine with aspartic acid (N125D) at the position corresponding to position 125 in SEQ ID NO: 1 and/or the substitution of lysine with asparagine (K185N) at the position corresponding to position 185 in SEQ ID NO: 1. It will be understood in this regard that the term "and/or" with regard to the substitutions refers to the possibility that said substitutions are the sole substitution ("or") or that two or all three of these substitutions are present in combination with each other. A polypeptide having the amino acid sequence of SEQ ID NO: 1 but comprising the substitution N125D and K185N is also referred to herein as Amrose v2 (shown in SEQ ID NO: 3) while a polypeptide having the amino acid sequence of SEQ ID NO: 1 but comprising the substitution E16G is referred to herein as Amrose v3 (shown in SEQ ID NO: 5). A polypeptide having the amino acid sequence of SEQ ID NO: 1 but comprising all three substitutions E16G, N125D and K185N is also referred to herein as Amrose v4 (shown in SEQ ID NO: 7). A polypeptide having a substitution of glutamic acid with glycine at the position corresponding to position 16 (E16G) in SEQ ID NO: 1 is for example encoded by a nucleic acid molecule having the nucleotide sequence of SEQ ID NO: 2, with the exception that said nucleic acid molecule has at the positions corresponding to positions 48 to 50 of SEQ ID NO: 2 a nucleotide triplet selected from the group consisting of GGT, GGC, GGA and GGG. A polypeptide having a substitution of asparagine with aspartic acid at the position corresponding to position 125 (N125D) of SEQ ID NO: 1 is for example encoded by a nucleic acid molecule having the nucleotide sequence of SEQ ID NO: 2, with the exception that said nucleic acid molecule has at the positions corresponding to positions 375 to 377 of SEQ ID NO: 2 a nucleotide triplet selected from the group consisting of GAT and GAC. Furthermore, a polypeptide having a substitution of lysine with asparagine (K185N) at the position corresponding to position 185 in SEQ ID NO: 1 is for example encoded by a nucleic acid molecule having the nucleotide sequence of SEQ ID NO: 2, with the exception that said nucleic acid molecule has at the positions corresponding to positions 555 to 557 of SEQ ID NO: 2 a nucleotide triplet selected from the group consisting of AAT and AAC

Also encompassed by the present invention is a nucleic acid molecule encoding a polypeptide having at least 94% sequence identity to the polypeptide encoded by the nucleic acid molecule of (a) or (b), i.e. the nucleic acid molecule encoding the polypeptide described herein above. More preferably, the nucleic acid molecule encodes a polypeptide having at least 95% sequence identity to the polypeptide encoded by the nucleic acid molecule of (a) or (b), such as for example at least 96%, such as at least 97%, more preferably at least 98%, such as at least 98.5%, such as at least 99% and most preferably at least 99.5% sequence identity to the polypeptide encoded by the nucleic acid molecule of (a) or (b). Preferably, the nucleic acid molecule encoding a polypeptide having at least 94% sequence identity to the polypeptide encoded by the nucleic acid molecule of (b) is a nucleic acid molecule wherein (i) glutamic acid at the position corresponding to position 16 of SEQ ID NO: 1 is glycine; (ii) asparagine at the position corresponding to position 125 of SEQ ID NO: 1 is aspartic acid and/or (iii) lysine at the position corresponding to position 185 of SEQ ID NO: 1 is asparagine.

In accordance with the present invention, the term "% sequence identity" describes the number of matches ("hits") of identical amino acids of two or more aligned amino acid sequences as compared to the number of amino acid residues making up the overall length of the amino acid sequences (or the overall compared part thereof). In other terms, using an alignment, for two or more sequences or subsequences the percentage of amino acid residues that are the same (e.g., 94% or 95% identity) may be determined, when the (sub)sequences are compared and aligned for maximum correspondence over a window of comparison, or over a designated region as measured using a sequence comparison algorithm as known in the art, or when manually aligned and visually inspected. Preferred polypeptides in accordance with the invention are those where the described identity exists over a region that is at least about 15 to 25 amino acids in length, more preferably, over a region that is at least about 50 to 100 amino acids in length. More preferred polypeptides in accordance with the present invention are those having the described sequence identity over the entire length of the polypeptide of SEQ ID NO:1. Those having skill in the art will know how to determine percent sequence identity between/among sequences using, for example, algorithms such as those based on the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402), CLUSTALW computer program (Thompson Nucl. Acids Res. 2 (1994), 4673-4680) or FASTA (Pearson and Lipman, Proc. Natl. Acad. Sci., 1988, 85; 2444), as known in the art.
The NCBI BLAST algorithm is preferably employed in accordance with this invention. For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. Accordingly, all the polypeptides having the prescribed function and further having a sequence identity of at least 94% as determined with the NCBI BLAST program fall under the scope of the invention.

It is preferred that the polypeptide having at least 94% sequence identity does not comprise amino acid substitutions in one or more of the positions corresponding to positions 82, 147, 158 and 197 of SEQ ID NO:1, i.e. in the following positions corresponding to the respective positions of SEQ ID NO:1 no amino acid substitution(s) is/are present as compared to SEQ ID NO:1: (i) position 82; (ii) position 147; (iii) position 158; (vi) position 197; (v) positions 81 and 147; (vi) positions 82 and 158; (vii) positions 82 and 197; (viii) positions 147 and 158; (ix) positions 147 and 197; (x) positions 158 and 197; (xi) positions 82, 147 and 158; (xii) positions 82, 147 and 197; (xiii) positions 82, 158 and 197; more preferably it does not comprise amino acid substitutions in (xiv) all of the positions corresponding to positions 147, 158 and 197 of SEQ ID NO:1 and most preferably it does not comprise amino acid substitution in (xv) all four of these positions. Accordingly, a polypeptide of the invention that deviates from the sequence of SEQ ID NO:1 may deviate in any position except in one of positions 82, 147, 158 and 197, where it comprises glutamic acid (E), methionine (M), asparagine (N) and tyrosine (Y), respectively, more preferably it may deviate in any position except in positions 147, 158 and 197, and most preferably it may deviate in any position except in positions 82, 147, 158 and 197.
In a more preferred embodiment, the polypeptide comprising amino acid substitutions does additionally not comprise substitutions at the positions corresponding to positions 16, 125 and/or 185 of SEQ ID NO:1, i.e. in (a) position 16; (b) position 125; (c) position 185; (d) position 16 and 125; (e) position 16 and 185; (f) position 125 and 185; and (g) all three positions 16, 125 and 185, except for the substitutions E16G, N125D and K185N described herein above.
The combination of non-substituted positions as defined in (i) to (xv) with non-substituted positions (a) to (g) in all possible combinations is explicitly envisaged herein, i.e. (i) and (a), (i) and (b), (i) and (c), (i) and (d), (i) and (e), (i) and (f), and (i) and (g), (ii) and (a), (ii) and (b), (ii) and (c), (ii) and (d), (ii) and (e), (ii) and (f), and (ii) and (g), (iii) and (a), (iii) and (b), (iii) and (c), (iii) and (d), (iii) and (e), (iii) and (f), and (iii) and (g), (iv) and (a), (iv) and (b), (iv) and (c), (iv) and (d), (iv) and (e), (iv) and (f), and (iv) and (g), (v) and (a), (v) and (b), (v) and (c), (v) and (d), (v) and (e), (v) and (f), and (v) and (g), (vi) and (a), (vi) and (b), (vi) and (c), (vi) and (d), (vi) and (e), (vi) and (f), and (vi) and (g), (vii) and (a), (vii) and (b), (vii) and (c), (vii) and (d), (vii) and (e), (vii) and (f), and (vii) and (g), (viii) and (a), (viii) and (b), (viii) and (c), (viii) and (d), (viii) and (e), (viii) and (f), and (viii) and (g), (ix) and (a), (ix) and (b), (ix) and (c), (ix) and (d), (ix) and (e), (ix) and (f), and (ix) and (g), (x) and (a), (x) and (b), (x) and (c), (x) and (d), (x) and (e), (x) and (f), and (x) and (g), (xi) and (a), (xi) and (b), (xi) and (c), (xi) and (d), (xi) and (e), (xi) and (f), and (xi) and (g), (xii) and (a), (xii) and (b), (xii) and (c), (xii) and (d), (xii) and (e), (xii) and (f), and (xii) and (g), (xiii) and (a), (xiii) and (b), (xiii) and (c), (xiii) and (d), (xiii) and (e), (xiii) and (f), and (xiii) and (g), (xiv) and (a), (xiv) and (b), (xiv) and (c), (xiv) and (d), (xiv) and (e), (xiv) and (f), and (xiv) and (g), (xv) and (a), (xv) and (b), (xv) and (c), (xv) and (d), (xv) and (e), (xv) and (f), and (xv) and (g).

In accordance with the present invention, it is required that the polypeptide having at least 94% sequence identity has a fluorescence emission activity at a wavelength of at least 630 nm, as defined herein above. Accordingly, it will be appreciated that sequence modifications of the polypeptide of the invention that lead to a total or partial loss of this function are not encompassed by the present invention. A partial loss, as referred to herein, refers to a reduction in fluorescence emission activity at a wavelength of at least 630 nm by more than 50%, preferably more than 60%, such as more than 70%, more preferably more than 80% and even more preferably 90%, such as 95% and most preferably 99%. It is preferred that the polypeptide having at least 94% sequence identity has the same or even an increased fluorescence emission activity at a wavelength of at least 630 nm as the polypeptide of SEQ ID NO:1.

The term "degenerate" in accordance with the present invention refers to the redundancy of the genetic code. Degeneracy results because there are more codons than encodable amino acids. For example, if there were two bases per codon, then only 16 amino acids could be coded for (4²=16). Because at least 21 codes are required (20 amino acids plus stop), and the next largest number of bases is three, then 4³ gives 64 possible codons, meaning that some degeneracy must exist. As a result, some amino acids are encoded by more than one triplet, i.e. by up to six triplets. The degeneracy mostly arises from alterations in the third position in a triplet. This means that nucleic acid molecules having a different sequence than that specified above, but still encoding the same polypeptide lie within the scope of the present invention.

In accordance with the present invention, highly stable, bright fluorescent protein variants are provided that are useable with standard lasers, that are non-cytotoxic and that function in a variety of research model systems including higher eukaryotic and vertebrate cells. To develop these fluorescent proteins, a variant of the wild type eqFP578 (sea anemone *Entacmaea quadricolor*) denoted eqFP615 (Evrogen JSC) with a 559nm absorption peak and a 615nm emission peak was evolved into the fluorescent protein Amrose with a 606nm absorption peak and a 645nm emission peak. While the shifts of the peaks are significant, it was surprisingly found that Amrose (a portmanteau of American rose) has an off peak ability to absorb and emit into the optical window that allows for deep tissue imaging, i.e. Amrose remains bright when excited at 633nm and demonstrates functionality in the whole mouse imaging model at 670nm. Even more, emission of the fluorescent protein of the present invention has been detected up to a wavelength of 850nm. These impressive attributes facilitate the investigation of metastasis and tumor localization, cell migration, embryogenesis and other studies involving deep-tissue imaging.

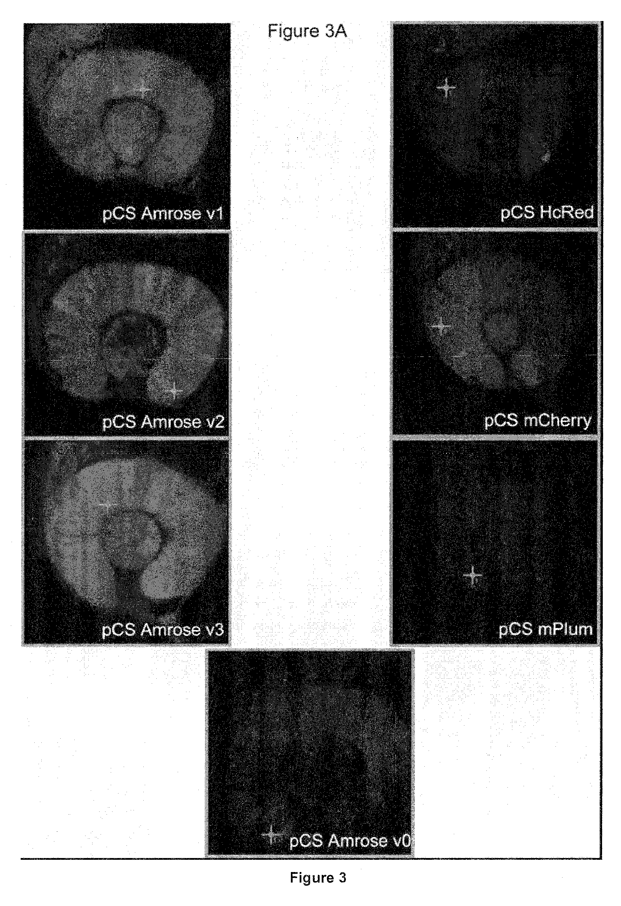

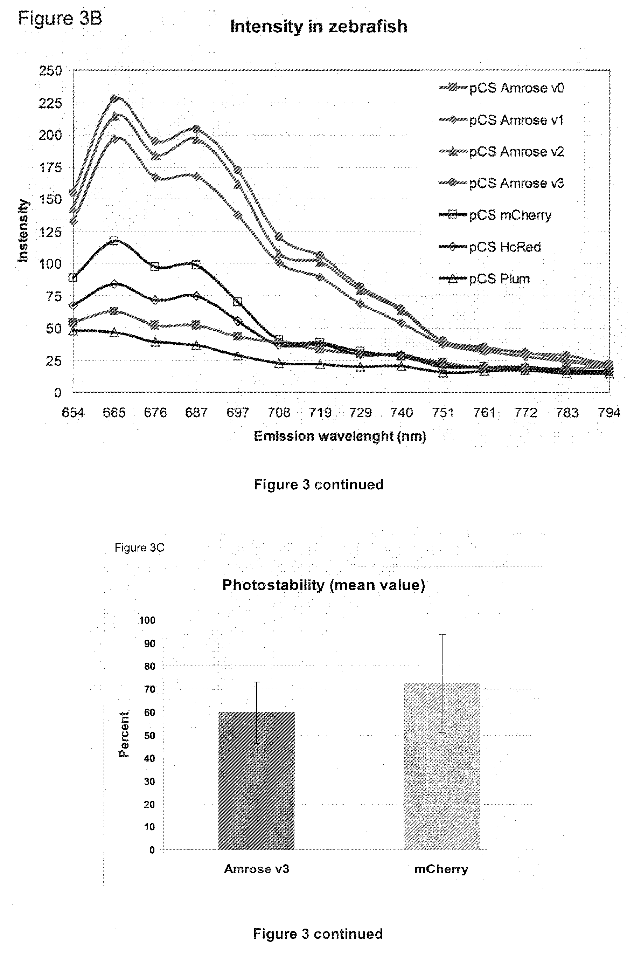

The relevant mutations in Amrose can be characterized via its evolutionary tree. Four mutations are shared by Amrose Variant 1 and subsequent variants. L147M was a very early mutation as was H197Y. Sequencing of sorts 13, 14 and 15 revealed that S158N appeared by sort 13. The fourth mutation, Q82E, appeared by sort 15 and coincided with a pronounced color change in the cells from more purple to more blue. Reversion of this mutation (v0) revealed its importance in both brightness and the far red spectral shift of Amrose in higher eukaryotes (see Figure 3A, 3B). Variant 2 has additionally K185N and N124D which appeared following sort 15. The third major variant contains the E16G mutation which appears to be a highly significant variation from Variant 1 in the zebrafish model. From this, and absent crystallographic data for Amrose specific variants, the inventors hypothesize, without wishing to be bound by theory, that Q82E, N125D and K185N as well as E16G significantly have either a role in the cis-trans isomerization profile and/or chromophore packing configurations previously described for the related fluorescent protein mKate (Pletnev, et. al., 2004) and/or in side chain hydrogen bonds as described for mNeptune (Lin, et. al., 2009).

Apart from their use in research, e.g. as reporter proteins, the fluorescent proteins of the present invention find further use, for example, when embedded in polymers or supported by stabilizers. In this form, the fluorescent proteins of the invention may be incorporated into toys that glow red in the dark when excited, equipment for fish tanks, they may be incorporated into target walls for laser writing or red-glowing cosmetics (e.g. hair gel) or may be employed as wavelength converting paint additive or medical products e.g. skin/wound cream (Photomed Laser Surg. 2005 Aug;23(4):355-61. Exact action spectra for cellular responses relevant to phototherapy. Karu TI, Kolyakov SF). Other medical uses include, without being limiting, labeling of intracellular pathogens like *H. pylori* and visualizing its pathogenic life cycle, or labeling of serum proteins and visualizing the vascular system, as well as labeling and visualizing neurons and RNA (Paige, JS, Wu, KY and Jaffrey SR; RNA Mimics of Green Fluorescent Protein; Science (2011) 333:642-646).

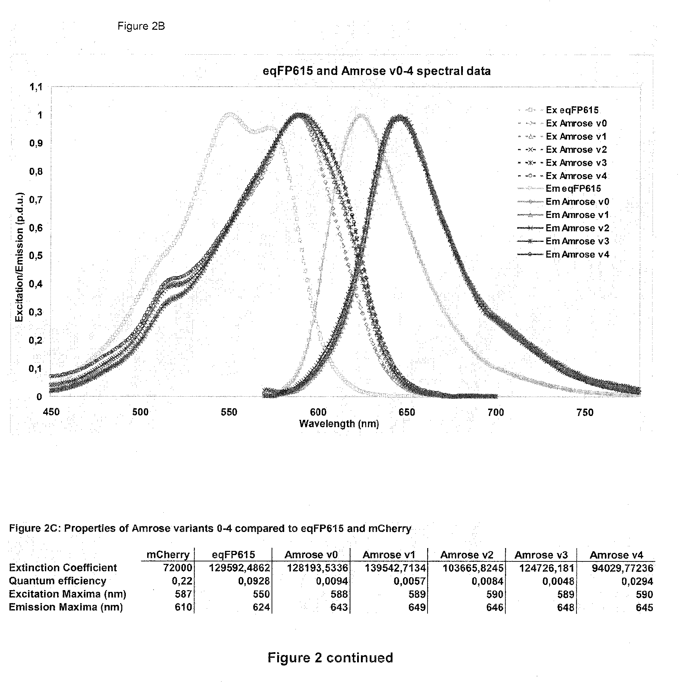

The fluorescent proteins of the present invention can advantageously be used in FRET experiments. This is possible due to a reduced absorption/excitation activity at wavelengths at which the most commonly employed fluorescent proteins, the green fluorescent proteins, are excited. For example, the naturally occurring GFP from *A. victoria* has a major excitation peak at a wavelength of 395nm and a minor one at 475nm. As is shown in Figure 2B, almost no absorption activity was observed for the Amrose polypeptides of the invention at e.g. 460nm. Consequently, the polypeptide of the present invention can be employed in studies, such as e.g. FRET, in which multiple, different fluorescent proteins are used simultaneously. Although such simultaneous studies are already available in the art, the fluorescent proteins employed so far suffer from the disadvantage of giving an unspecific (i.e. background) signal after excitation at the lower wavelength required for excitation of e.g. GFP. The polypeptide of the present invention overcomes these drawbacks and, consequently, represents an improved fluorescent protein for such parallel studies. To the inventor's best knowledge, no far-red fluorescent proteins having no or substantially no absorption/excitation activity at wavelengths of 488nm or less have been described in the art so far. To give one specific example, the fluorescent proteins of the invention could be paired with LSS-mKate2 (460/605) to make such a FRET pair. In addition, and as shown in the appended examples, an exceptionally high Stokes shift could be observed, i.e. the wavelength for excitation and emission are far apart. Thus, the background signal often caused by filter leakage when using overlapping excitation and emission spectra is substantially reduced.

The present invention further relates to a vector comprising the nucleic acid molecule of the invention.

Preferably, the vector is a plasmid, cosmid, virus, bacteriophage or another vector used conventionally e.g. in genetic engineering.

The nucleic acid molecule of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen), lambda gt11, pJOE, the pBBR1-MCS series, pJB861, pBSMuL, pBC2, pUCPKS, pTACT1, pRSET A, B and C (Life Technologies) and vectors compatible with expression in mammalian cells like E-027 pCAG Kosak-Cherry (L45a) vector system, pShuttle-CMV and pCAG (both from Addgene), pREP (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, Okayama-Berg cDNA expression vector pcDV1 (Pharmacia), pRc/CMV, pcDNA1, pcDNA3 (Invitrogene), pSPORT1 (GIBCO BRL), pGEMHE (Promega), pLXIN, pSIR (Clontech), pIRES-EGFP (Clontech), pEAK-10 (Edge Biosystems) pTriEx-Hygro (Novagen), pGreenPuro™ shRNA Cloning and Expression Lentivector (System Biosciences) and pCINeo (Promega). Examples for plasmid vectors suitable for Pichia pastoris comprise e.g. the plasmids pAO815, pPIC9K and pPIC3.5K (all Invitrogen). Another vector suitable for expressing proteins in xenopus embryos, zebrafish embryos as well as a wide variety of mammalian and avian cells is the multipurpose expression vector pCS2+.
The nucleic acid molecule of the present invention referred to above may also be inserted into vectors such that a translational fusion with another nucleic acid molecule is generated. The other nucleic acid molecules may e.g. encode a protein that increases the solubility and/or facilitates the purification of the protein encoded by the nucleic acid molecule of the invention or a protein of interest that is to be observed by fluorescence imaging. Non-limiting examples of such vectors include pET32, pET41, pET43. The vectors may also contain an additional expressible polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosettaâ.
For vector modification techniques, see Sambrook and Russel "Molecular Cloning, A Laboratory Manual", Cold Spring Harbor Laboratory, N.Y. (2001). Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M 13 origins of replication.
The coding sequences inserted in the vector can e.g. be synthesized by standard methods, or isolated from natural sources. Ligation of the coding sequences to transcriptional regulatory elements and/or to other amino acid encoding sequences can be carried out using established methods. Such regulatory sequences are well known to those skilled in the art and include, without being limiting, regulatory sequences ensuring the initiation of transcription, internal ribosomal entry sites (IRES) (Owens, Proc. Natl. Acad. Sci. USA 98 (2001), 1471-1476) and optionally regulatory elements ensuring termination of transcription and stabilization of the transcript. Non-limiting examples for regulatory elements ensuring the initiation of transcription comprise a translation initiation codon, enhancers such as e.g. the SV40-enhancer, insulators and/or promoters, such as for example the cytomegalovirus (CMV) promoter, SV40-promoter, RSV-promoter (Rous sarcome virus), the lacZ promoter, chicken beta-actin promoter, CAG-promoter (a combination of chicken beta-actin promoter and cytomegalovirus immediate-early enhancer), the gai10 promoter, human elongation factor 1α-promoter, AOX1 promoter, GAL1 promoter CaM-kinase promoter, the lac, trp or tac promoter, the lacUV5 promoter, the autographa californica multiple nuclear polyhedrosis virus (AcMNPV) polyhedral promoter or a globin intron in mammalian and other animal cells. The lac promoter is a typical inducible promoter, useful for prokaryotic cells, which can be induced using the lactose analogue isopropylthiol-b-D-galactoside ("IPTG"). Non-limiting examples for regulatory elements ensuring transcription termination include the V40-poly-A site, the tk-poly-A site or the SV40, lacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing, nucleotide sequences encoding secretion signals or, depending on the expression system used, signal sequences capable of directing the expressed polypeptide to a cellular compartment. Moreover, elements such as origin of replication, drug resistance gene, regulators (as part of an inducible promoter) may also be included.
An expression vector according to this invention is capable of directing the replication and the expression of the nucleic acid molecule of the invention and the polypeptide encoded thereby.

The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the cells are grown in selective medium and the cells with the highest resistance are selected. Expression vectors will preferably include at least one selectable marker. Such markers include dihydrofolate reductase, G418 or neomycin resistance, puromycin, blasticidin as well as mycophenolic acid for eukaryotic cell culture and tetracycline, kanamycin or ampicillin resistance genes for culturing in *E. coli* and other bacteria.

Alternatively, expression of the fluorescent protein of the invention as part of the construct can be employed for the identification and isolation of the transfected cells, e.g. by cell sorting employing flow cytometry. For example, such fluorescent technology may be used to select for cells expressing a co-expressed transgene (for example: promoter - gene of interest - IRES - Amrose). This eliminates the need to expose the cell lines to hygromycin and other toxic selection markers. In particular for cell lines that will be used in animal models, such as mouse models, it is often advantageous to avoid the use of such markers. This is for example advantageous for labeling of tumor cells, which may then be introduced into an animal for use in anti-tumor drug screening. The live animal can be monitored via the fluorescent signal without the need to sacrifice the animal, thereby saving animals as well as costs.

The nucleic acid molecules of the invention as described herein above may be designed for direct introduction or for introduction via electroporation (using for example Multiporator (Eppendorf) or Genepulser (BioRad)), PEI (Polysciences Inc. Warrington, Eppelheim), Ca²⁺-mediated transfection or via liposomes (for example: "Lipofectamine" (Invitrogen)), non-liposomal compounds (for example: "Fugene" (Roche)), liposomes, phage vectors or viral vectors (e.g. adenoviral, retroviral, lentiviral) into cells. Additionally, baculoviral systems or systems based on Vaccinia Virus or Semliki Forest Virus can also be used as vector in eukaryotic expression system for the nucleic acid molecules of the invention. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook and Russel "Molecular Cloning, A Laboratory Manual", Cold Spring Harbor Laboratory, N.Y. (2001) and Ausubel et al., Current Protocols in Molecular Biology, Green Publishing Associates and Wiley Interscience, N.Y. (2001).

The present invention further relates to a non-human host transformed with the vector of the invention.

Non-human hosts according to the present invention can be single cells or multi-cellular organisms.

Suitable prokaryotic hosts comprise e.g. bacteria of the species *Escherichia, Streptomyces, Salmonella* or *Bacillus.* Suitable eukaryotic host cells are e.g. yeasts such as *Saccharomyces cerevisiae* or *Pichia pastoris* or chicken cells, such as e.g. DT40 cells. Insect cells suitable for expression are e.g. Drosophila S2 or Spodoptera Sf9 cells. Suitable zebrafish cell lines include, without being limiting, ZFL, SJD or ZF4. Mammalian host cells that could be used include, human Hela, HEK293, H9 and Jurkat cells, mouse NIH3T3 and C127 cells, COS 1, COS 7 and CV1, quail QC1-3 cells, mouse L cells, mouse sarcoma cells, Bowes melanoma cells and Chinese hamster ovary (CHO) cells. Also within the scope of the present invention are primary mammalian cells or cell lines. Primary cells are cells which are directly obtained from an organism. Suitable primary cells are, for example, mouse embryonic fibroblasts (MEF), mouse primary hepatocytes, cardiomyocytes and neuronal cells as well as mouse muscle stem cells (satellite cells) and stable, immortalized cell lines derived thereof (for example hTERT immortalized cells). Alternatively, the recombinant protein of the invention can be expressed in stable cell lines that contain the gene construct integrated into a chromosome.
Appropriate culture media and conditions for the above described host cells are known in the art.

Transgenic non-human animals as hosts transfected with and/or expressing the nucleic acid molecule of the present invention also lie within the scope of the invention. In a preferred embodiment, the transgenic animal is a fish, such as e.g. zebrafish or a mammal, e.g. a mouse, rat, hamster, cow, cat, pig, dog, horse, rabbit or monkey.

Transgenic plants as hosts transfected with and/or expressing the nucleic acid molecule of the present invention also lie within the scope of the present invention. The skilled person is aware of suitable means and methods to ensure expression of transgenes in plants. Co-expression of the fluorescent proteins of the present invention in a transgenic plant is of particular interest for the production of proteins of interest, as the fluorescent protein may aid in locating the site of expression as well as in the purification of the proteins of interest from the plant tissue (see e.g. Ckurshumova et al. 2011; Glow in the Dark: Fluorescent Proteins as Cell and Tissue-Specific Markers in Plants; Mol. Plant (2011) doi: 10.1093/mp/ssr059).

The non-human animal or plant in accordance with this embodiment can be used in accordance with the invention e.g. in a method for identification of compounds, described herein below.

A method for the production of a transgenic non-human animal, such as e.g. a transgenic zebrafish, has been described in the appended examples. Other methods include for example methods for the production of transgenic mice or other mammals, which usually comprise introduction of the nucleic acid molecule or targeting vector of the present invention into a germ cell, an embryonic cell, stem cell or an egg or a cell derived therefrom. Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., Southern blots with an appropriate probe. A general method for making transgenic non-human animals is described in the art; see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), the AK-7 line (Soriano P. Development (1997) 124:2691-2700). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born, e.g. as chimeric mice. The resultant transgenic mice are chimeric for cells having either the recombinase or reporter loci and are backcrossed and screened for the presence of the correctly targeted transgene(s) by PCR or Southern blot analysis on tail biopsy DNA of offspring so as to identify transgenic mice heterozygous for either the recombinase or reporter locus/loci.

Furthermore, the present invention also relates to a method of producing a polypeptide having a fluorescence emission activity at a wavelength of at least 630 nm, the method comprising culturing the non-human host of the invention under suitable conditions and isolating the thereby produced polypeptide having a fluorescence emission activity at a wavelength of at least 630 nm. Preferably, the method is carried out using host cells, such as e.g. bacterial cells.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be over-expressed. In general, the skilled person is also aware that these conditions may have to be adapted to the needs of the host and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycine. The cells can be kept e.g. at 37 °C or at 41°C for DT40 chicken cells, in a 5% CO2, water saturated atmosphere.
Suitable media for insect cell culture is e.g. TNM + 10% FCS or SF900 medium. Insect cells are usually grown at 27 °C as adhesion or suspension culture.
Suitable expression protocols for eukaryotic or vertebrate cells are well known to the skilled person and can be retrieved e.g. from Sambrook and Russel "Molecular Cloning, A Laboratory Manual", Cold Spring Harbor Laboratory, N.Y. (2001).

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook and Russel "Molecular Cloning, A Laboratory Manual", Cold Spring Harbor Laboratory, N.Y. (2001).

The present invention further relates to a polypeptide comprising the amino acid sequence encoded by the nucleic acid molecule of the invention or producible or produced by the method of the invention.

Accordingly, this embodiment relates to a polypeptide comprising any one of the amino acid sequences recited herein, such as for example the amino acid sequences represented by SEQ ID NOs: 1, 3 or 5.

Furthermore, the present invention relates to a fusion protein comprising the polypeptide of the invention.

In addition to the amino acid sequence of the far-red fluorescent protein variants of the present invention, a fusion protein according to the present invention contains at least one additional, heterologous amino acid sequence. Often, but not necessarily, these additional sequences will be located at the N- or C-terminal end of the polypeptide. It may e.g. be convenient to initially express the polypeptide of the invention as a fusion protein from which the additional amino acid residues can be removed, e.g. using a proteinase capable of specifically cleaving the additional amino acid residues from the polypeptide of the present invention. The far-red fluorescent protein variants of the present invention can also be fused to a protease cleavage sequence and a quencher that, when cleaved, allows identification by a corresponding increase in fluorescence.

Such additional heterologous sequences may e.g. be tags, i.e. sequences that help in the expression or purification of the polypeptides referred to in the present invention.

Accordingly, in one embodiment of the fusion protein of the invention, the polypeptide of the invention is fused to a His-tag, a Strep-tag, a GST-tag, a TAP tag, biotin, an HA tag or a signal sequence for intra- or extracellular targeting. Signal sequences for intra- or extracellular targeting include, without being limiting, nuclear localization signals, signals to retain a protein in the endoplasmatic reticulum, signals to target a protein to peroxisomes, secretion signals or signals for trans-Golgi-network sorting.

These sequences are conveniently used to facilitate expression and/or purification of proteins. Examples for a signal sequence for intracellular targeting such as e.g. nuclear localization signals include for example the sequence PPKKKRKV; examples for signals to retain a protein in the endoplasmatic reticulum include e.g. the sequence KDEL and example for signals for peroxisomes include e.g. SKL.

Alternatively, the far-red fluorescent protein of the invention may serve as a tag-sequence when attached to a particular protein of interest, such as e.g. a signaling protein or a protein, the presence or localization of which in cells and/or tissues is to be investigated. In that case, the heterologous sequence is the protein of interest and the fluorescent protein of the invention serves as a tag.

Exemplary proteins are those forming the cytoskeleton or proteins forming part of a signal transduction pathway or membrane proteins binding to one or more ligands, e.g. for studying the effects of drugs, receptor-ligand binding, proteins tagged for RNAi studies, protease activity, cell differentiation or proteins attached to nano-probes and quantum dots. Useful assays for the far-red fluorescent protein variants of the present invention are e.g. FRET assays or two-hybrid assays. In general, the far-red fluorescent protein variants of the present invention and fusion proteins comprising them are useful as biomarkers and in diagnostics.

The present invention further relates to a diagnostic composition comprising at least one of (a) the nucleic acid molecule of the invention; (b) the vector of the invention; (c) the polypeptide of the invention; and/or (d) the fusion protein of the invention.

In accordance with the present invention, the term "diagnostic composition" relates to compositions for diagnosing individual patients for the presence or absence of particular cells, nucleic acid molecules, proteins or peptides, usually in an assessment of a potential therapeutic intervention. For example, hematological profiling in combination with flow cytometry may employ the fluorescent proteins of the invention. The diagnostic composition of the invention comprises at least one of the compounds recited above and may further comprise appropriate buffer(s), and enzymes such as reverse transcriptase, thermostable polymerases etc.. The diagnostic compositions may be packaged in a container or a plurality of containers, optionally with instructions for use.

The present invention also relates to a method of detecting the expression of a gene of interest in a sample, the method comprising providing a light stimulus to the sample and detecting the fluorescence emitted by a polypeptide encoded by the nucleic acid molecule of the invention, wherein said nucleic acid molecule is (a) operably linked to a promoter controlling said gene of interest; or (b) fused to said gene of interest.

Upon provision of the light stimulus, for example a stimulus at a wavelength of approx. 633 nm, the far-red fluorescent protein of the invention is excited and subsequently can emit a fluorescent signal.

The detection of fluorescence is well known in the art. The experimental set up will dictate the actual filter to be used and can vary from experiment to experiment. Generally, detection is carried out at a wavelength longer than the excitation wavelength employed. For the far-red fluorescent proteins of the present invention, detection is possible up to 850 nm, as described herein above.

Four essential elements of fluorescence detection systems can be identified: 1) an excitation source, 2) a fluorophore, 3) wavelength filters to isolate emission photons from excitation photons and 4) a detector that registers emission photons and produces a recordable output, usually as an electrical signal or a photographic image. Regardless of the application, compatibility of these four elements is essential for optimizing fluorescence detection.

Fluorescence instruments are primarily of four types, each providing distinctly different information: Spectrofluorometers and microplate readers measure the average properties of bulk (µL to mL) samples. Fluorescence microscopes resolve fluorescence as a function of spatial coordinates in two or three dimensions for microscopic objects (less than ∼0.1 mm diameter). Fluorescence scanners, including microarray readers, resolve fluorescence as a function of spatial coordinates in two dimensions for macroscopic objects such as electrophoresis gels, blots and chromatograms. Flow cytometers measure fluorescence per cell in a flowing stream, allowing subpopulations within a large sample to be identified, quantified and isolated. Other types of instrumentation that use fluorescence detection include capillary electrophoresis apparatus, DNA sequencers and microfluidic devices. Each type of instrument produces different measurement artifacts and has different demands on the fluorescent probe. For example, although photo-bleaching is often a significant problem in fluorescence microscopy, it is not a major impediment in flow cytometry or DNA sequencing because the dwell time of individual cells or DNA molecules in the excitation beam is short. Additional methods for determining fluorescence, in particular far red fluorescence, include CCD-based fluorescence tomography and multispectral optoacoustic (photoacoustic) tomography (MSOT).

In accordance with the present invention and depending on the demands and possibilities of the respective methods, detection of the fluorescent signal within a sample can for example be carried out as a measurement of the overall fluorescence in the sample or, alternatively, as the determination of the fluorescent signal within the sample. A sample can be any matter potentially containing the molecule of interest. Preferred samples are liquids, e.g. cellular extracts or supernatants of cell cultures, or samples comprising living or dead cells in single form or present as a tissue or organ.

In accordance with this embodiment, the nucleic acid molecule encoding the far-red fluorescent protein of the invention may serve as a reporter gene. A reporter gene is a gene that can be operably linked to a gene of interest or, alternatively, to a promoter controlling the gene of interest in cell culture, animals or plants. Reporter genes are e.g. used to determine whether the gene of interest has been taken up by or is expressed in the cell or organism population or whether the gene of interest naturally present in the cell or organism is expressed. In this regard, it is important to use a reporter gene that is not endogenously expressed in the cell or organism under study, since the expression of the reporter serves as a marker. The nucleic acid of the present invention encodes far-red fluorescent protein variants suitable as reporter genes with advantageous properties as described above.
One approach to introduce a reporter gene into an organism is to place the reporter gene and the gene of interest into the same DNA construct to be inserted into the cell or organism, most often a plasmid. In this case the reporter gene is directly attached to the gene of interest to create a gene fusion. The two genes are under the control of the same promoter and are transcribed into a single messenger RNA molecule (mRNA). The mRNA is then translated into protein. It is important that both proteins are able to properly fold into their active conformations and interact with their substrates despite being fused. For example, when building the DNA construct, a segment of DNA coding for a flexible polypeptide linker region is often included so that the reporter gene and the gene product of interest will only minimally interfere with one another. Methods to determine correct protein folding and activity are well-known in the art. Another approach to introduce a reporter gene is to operably link it in a bicistronic message to the gene of interest via an IRES sequence under the direction of a single promoter as previously described above.

In another approach, gene targeting methods are applied to exchange a gene of interest for the fluorescent protein of the invention while ensuring that the fluorescent protein is under the control of the endogenous promoter of said gene of interest. This approach results in the expression of the fluorescent protein instead of the protein of interest, which can subsequently be detected and provides information about the expression of the gene of interest.

In addition, the present invention also relates to a method of detecting the activity of a promoter of interest in a sample, the method comprising providing a light stimulus to the sample and detecting the fluorescence emitted by the polypeptide encoded by the nucleic acid molecule of the invention, wherein said nucleic acid molecule is operably linked with said promoter of interest.

The analysis of the activity of promoters, which has been proven to be a highly sensitive and time-saving technique, is widely used e.g. in the study of transcriptional regulation. Reporter genes can also be used to assay the activity of a particular promoter in a cell or organism. In this case the reporter gene is simply placed under the control of the target promoter, as described above, and the reporter gene product's activity is quantitatively measured. The results are normally reported as a relative value compared to the activity under a consensus promoter known to induce strong gene expression. In addition, random introduction of the far-red fluorescent proteins into a chromosome can be used to identify promoters.

Promoter and reporter assays are commonly known in the art (see e.g. Lewin, "Genes IX", Jones & Bartlett Publishers, (2007) or Sambrook and Russel "Molecular Cloning, A Laboratory Manual", Cold Spring Harbor Laboratory, N.Y. (2001)).

The present invention further relates to a method of detecting the presence of a protein of interest, the method comprising contacting a sample with the fusion protein of the invention, wherein said fusion protein comprises a polypeptide specifically binding to the protein of interest, providing a light stimulus to the sample and detecting the fluorescence emitted by the fusion protein.

This method of the invention can be carried out using for example fluorescence activated cell sorting (FACS) scans and preparative FACS sorting.

Contacting a sample in accordance with the present invention refers to bringing the sample into contact with the fusion protein of the invention. Said contacting comprises bringing both components into close vicinity as well as e.g. transforming or transfecting cells with a nucleic acid molecule encoding the fusion protein of the invention in order to express the fusion protein in the cells.

Specifically binding in accordance with the present invention refers to the property of certain proteins to have an affinity for specific proteins, i.e. target proteins, which is greater by the factor of at least 10, preferably at least 100, more preferably at least 1000 and most preferably at least 10000 as compared to the affinity to proteins unrelated to the target protein. These proteins can e.g. be natural interaction partners of the target protein including proteins interacting in the course of signal transduction pathways or in context with structural proteins or antibodies.

The present invention furthermore relates to a method of detecting the localization of a polypeptide of the invention or a fusion protein of the invention in a cell or tissue, the method comprising providing said cell or tissue with a light stimulus and detecting the location of the polypeptide or fusion protein by (a) detecting the fluorescence emitted by the polypeptide or fusion protein upon excitation by the light stimulus; and/or (b) detecting the temperature change produced by the polypeptide or fusion protein upon absorption of the light stimulus.

Upon provision of the light stimulus, the far-red fluorescent proteins of the invention are either excited and subsequently emit a fluorescent signal as described above, or they absorb the energy provided by the stimulus, which may be measured by detecting the temperature change associated with this absorption.

Detecting the temperature change of the polypeptide or fusion protein upon absorption of the light stimulus is also known in the art under the term "optoacoustic methods", and is based on absorption of the stimulus, which leads to a local heating and accompanying local expansion. This local expansion leads to ultrasonic pressure waves that can be recorded using high frequency pressure sensors (Razansky *et al.* 2011; Ntziachristos and Razansky, 2010)

The above methods of the present invention are also suitable in the identification of compounds influencing e.g. the expression of genes of interest, the location of proteins of interest or the activity of such proteins of interest. Said compounds may be contacted with a cell comprising the nucleic acid of the invention, e.g. fused to a gene of interest or a regulatory sequence. Afterwards, the expressed polypeptide of the invention is excited in a cell contacted with the compound(s) and in a cell which has not been contacted with said compound. The expression or location of the polypeptide of the invention in both cells is compared, wherein a difference observed in the expression or location in both cells indicates that the compound has an influence on the expression of target genes and/or the location or activity of target proteins.

The present invention further relates to a kit comprising at least one of (a) the nucleic acid molecule of the invention; (b) the vector of the invention; (c) the host of the invention; (d) the polypeptide of the invention; or (e) the fusion protein of the invention.

The various components of the kit may be packaged in one or more containers such as one or more vials. The vials may, in addition to the components, comprise preservatives or buffers for storage and, optionally, instructions for use.

Unless otherwise defined, all technical and scientific terms used herein have the same meaning as commonly understood by one of ordinary skill in the art to which this invention belongs. In case of conflict, the patent specification, including definitions, will prevail.

The figures show:
**Figure 1** **Sequence comparison.** Sequence comparison (BioEdit) of selected published variants of the original eqFP578 to that of the five Amrose variants (v0-v4).
**Figure 2** **Spectral comparison.**
   **A)** Excitation and emission profile of Amrose v1 using whole cell protein extract from DT40 cells versus that of *E.coli* produced purified protein using pRSET vector (Invitrogen). Procedure defined unit = p.d.u.
   **B)** Excitation spectrum of the original eqFP615 and those of Amrose v0-v4 taken from *E.coli* produced purified protein using pRSET vector (Invitrogen). Procedure defined unit = p.d.u.
   **C)** Spectral properties of Amrose variants 0-4 compared to eqFP615 and mCherry.
**Figure 3** **Comparison of Amrose variants in the zebrafish model.**
   **A)** Amrose v0-v3 variants, as well as HcRed, mCherry and mPlum, visualized in zebrafish 24 hours after 100ng/µl mRNA injection using 633 nm excitation and scanning the emission spectrum. The crosshairs indicate where the measurement took place.
   **B)** Intensity as recorded in zebrafish (crosshairs show where measurement took place) at an excitation wavelength of 633 nm and scanned for emission at 11 nm intervals from 654 nm to 794 nm. Procedure defined unit = p.d.u.
   **C)** Photostability of Amrose v3 compared to that of mCherry in zebrafish. Each was tested in triplicate, excitation of 633nm at 100% power and a time-course of 150 scans with intensity loss calculated from the comparison of 10 spots between the first and last scans (p-value of 0.00377).

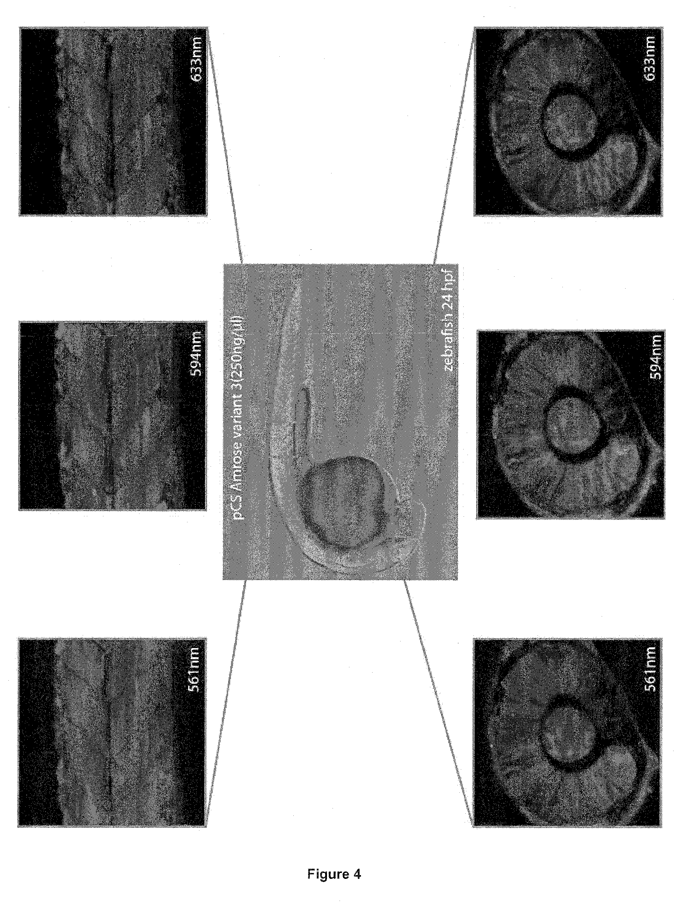

**Figure 4** **Visualization of whole zebrafish embryo at 561, 594 and 633 nm.** Amrose v3 visualized in zebrafish 24 hours after 250ng/µl mRNA injection at 561 nm, 594 nm and 633 nm excitation wavelengths. The boxes show where the measurement took place.

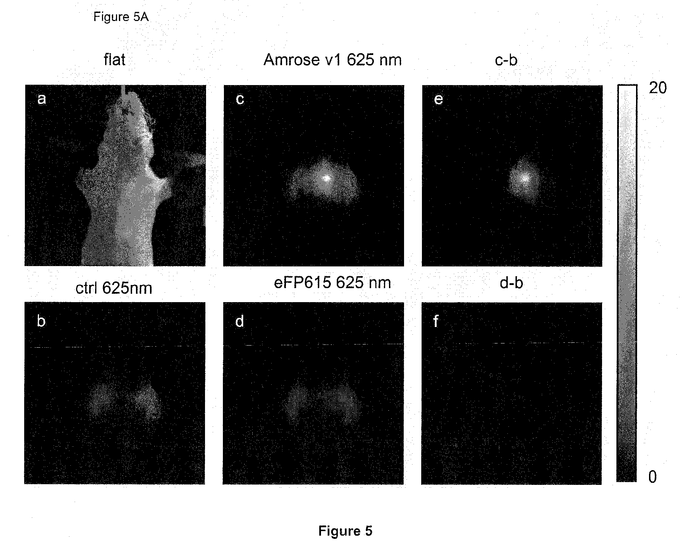

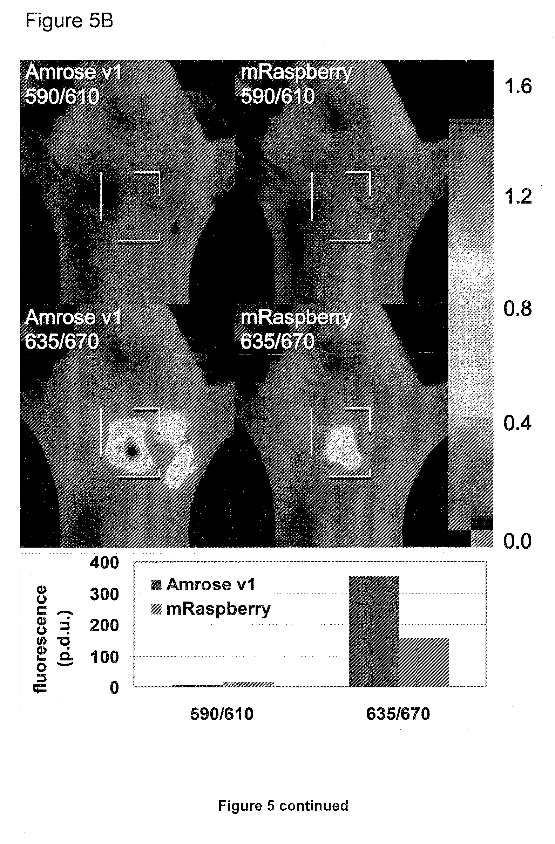

**Figure 5** **Whole-body imaging**
   **A)** Comparing Amrose v2 to eqFP615. Fluorescence images of FPs samples in the esophagous using 625 nm fixed excitation across mouse: (a) white light, (b) background, (c) Amrose v2 labeled DT40 cells (d) eqFP615 labeled DT40 cells (e) Amrose v2 minus background and (f) eqFP615 minus background.
   **B)** Comparing Amrose v1 to Raspberry. Fluorescence images of FPs samples in the esophagous using 2 different sets of excitation/emission wavelengths (590/610 and 635/670) overlaid on black and white images. Pixel values are in arbitrary units. Colorbar displays the transparency function. Bar chart - Comparison of accumulated FP intensity over a region of interest (dotted square) at the peak wavelength. Procedure defined unit = p.d.u.

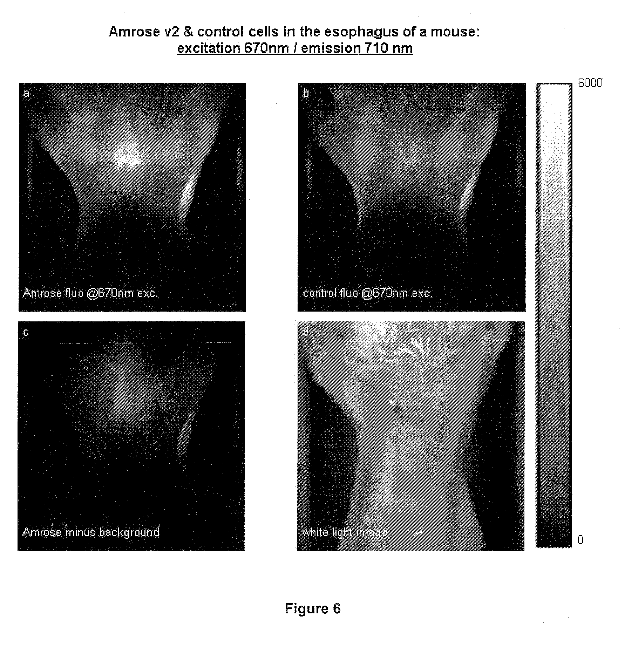

**Figure 6** **Whole-body imaging using Amrose v2 in FMT.** Amrose v2 expressing DT40 cells imaged with an Fluorescence Molecular Tomography (FMT) system which allows for tomographic reconstruction of the tube using 670 nm excitation and 710 nm emission.

The examples illustrate the invention:

### Example 1: Material and Methods

### Cell Culture

DT40 cells were cultured in chicken medium (DMEM/F-12 supplemented with 10% fetal bovine serum, 1 % chicken serum, 2mM L-glutamine, 0.1mM b-mercaptoethanol and penicillin/streptomycin) at 41°C in a 5% CO2 environment. To keep the cells vital, a cell density of 0.5-1.5 *10⁶ cells per ml medium was maintained and preparative sorts were performed at approximately two week intervals.

AID expression in the DT40 clones (a prerequisite for continuous hypermutation) was assured by transfection of a floxed AID-IRES (internal ribosome entry site)-gpt (guanine phosphoribosyl transferase) bicistronic cassette and cultured in the presence of 0.5 mg/ml of mycophenolic acid for 3 days following each preparative FACS sort. This was done as the DT40 cell clone used expresses an inducible form of the Cre recombinase (a MerCreMer fusion protein) in order to excise the floxed AID and thereby stop mutational activity. However, leaky background activity of MerCreMer can lead to undesired excision of the floxed AID-gpt expression cassette during prolonged culture. To isolate single colony variants in a non-mutating background, the floxed AID expression cassette was excised. AID negative subclones of far red sort 20 (FR_S20) were generated by culturing the cells in chicken medium (CM) containing 20 nM 4-hydroxitamoxifen (SIGMA) for three days followed by two rounds of cell sorting for stabilization. The resulting cell population FR_S22 was subcloned to produce single cell mycophenolic acid sensitive colonies (AID excised) for sequence analysis. For subcloning, cells were plated on three 96 well plates in concentrations of 300, 100 and 30 cells per plate in a volume of 200µl CM per well. After one week of incubation, single colonies were picked. The subclones were tested in normal CM and CM containing 0.5µg/ml mycophenolic acid for their sensitivity to this drug. Cell clones that did not survive a 3 day mycophenolic acid treatment were assumed to be AID negative and candidates were further confirmed by PCR using the primer-set AI24/AI25 (5' cccagatcttgcttgtgaagtcttcttattgctg 3' / 5' cccgctagcgccaccatggacagcctcttgatgaagagga 3').

### Flow cytometry

Fluorescent activated cell sorts were performed using the FACSAria and the FACSStar PLUS (BD Biosciences). 1-3*10⁸ cells were spun down and resolved in 3-10 ml PBS (density of the cells has to be adjusted to the flow rate abilities of the cell sorter). Selection criteria for the preparative FACS sorts were increased fluorescence in APC (660/20BP) and APC-Cy7 filter (735LP-780/60BP) for sort 1 to 8 in order to shift fluorescence to the near infrared and to increase brightness. Excitation was achieved with a red laser (633 nm). Sort 9 to 20 had the additional criteria of decreased fluorescence in the FITC filter (502LP-530/30BP), which is thought to reduce residual green fluorescence and to shift fluorescence further to the infra-red. Excitation in this case is achieved at 488 nm with the blue laser. The upper 0.2% of cells with the highest intensity and emission in the red laser and lowest intensity and emission in the blue laser were gated for sorting.

### PCR, cloning and sequencing

The cloning and lg locus integration of eqFP615 was performed as previously described (Arakawa, et. al., 2008). Crude genomic DNA extracts of 78 AID-negative Amrose subclones were prepared by incubation of cell pellets of app. 1.5*10⁶ cells in Expand Long Template PCR system Buffer 2 (Roche) supplemented with 0.5% Tween20 and 0.1 mg/ml Proteinase-K. Incubation was carried out at 56°C for 50 min and 95°C for 10 min. The PCR amplification of the Amrose transgenes was performed with the Expand Long Template PCR system (Roche) using 1µl aliquots of the crude extracts and the primer pair rbc580/rbc271 (5' gtgtgttggaggtcgctgagtagtgcgcgagc 3' / 5' ggctgattatgatcctctagagtcg 3'). The reaction mix per sample consisted of 6.33µl H2O, 1µl buffer 1, 1µl Cresol Red, 0.2µl dNTP mix (10mM), 0.07µl polymerase mix, 0.2µl each forward/reverse primer (25µM stock) and 1µl template for a total reaction volume of 10µl. PCR conditions were: 2 min of initial incubation at 93°C, 35 cycles consisting of 10 s at 93°C, 30 s at 65°C and 5 min (plus an added 20 s per cycle) at 68°C and a final elongation step of 7 min at 68°C.
The amplicons were gel purified using the Qiagen gel purification kit according to manufacturer's protocol. 2µl aliquots of the purified PCR product were used for sequencing PCR with BigDye Terminator v3.1 Cycle Sequencing Kit (Applied Biosystems). The reaction mix per sample consisted of 0.5 µl DMSO, 4.1 µl DEPC H2O, 1 µl 5x buffer BDT, 2 µl BDT, 0.4 µl primer (25µM stock) and 2 µl template. The primers used for sequencing were rbc584 (5' agacgggtctgacatggattggacgaa 3') and rbc276 (5' ccgcccgggtcggcttcggtcggagccatggagatc 3'). PCR conditions were: 4 min of initial incubation at 96°, 45 cycles consisting of 30 sec at 95°, 20 sec at 50° and 4 min at 60°. Reactions were EDTA/ethanol precipitated, resuspended in chromatography grade H2O and analyzed on an ABI 3730 DNA Analyzer (Applied Biosystems).

### Protein analysis of optical properties

For whole cell extract (wce) measurements, 50 million DT40 cells expressing Amrose v1 (and control cells not expressing a FP) were pelleted via centrifugation. The supernatant was removed and the pellet was resuspended in 2 ml 1X PBS containing Complete Mini EDTA-free protease inhibitor cocktail (Roche). The resuspended cells were lysed via two cycles of freezing and thawing using liquid nitrogen. The lysate was cleared of cell debris by centrifugation and passing the supernatant containing the extracted proteins twice through 0.2 µm Minisart syringe filters (Sartorius Stedim). The wce was analyzed using a LS50B Luminescence Spectrometer (PerkinElmer).
For purified protein measurements, Amrose v0-v4 variants and epFP615 were cloned into pRSETa (Invitrogen) via the BamHl-Xhol restriction site. The resultant clones were sequenced for confirmation, then used to transform E. coli BL21 via heat-shock and grown to an OD600 of -0.6-0.8 in 200ml LB containing 50µg/ml ampicillin. Protein expression was induced with 1 mM isopropyl β-d-thiogalactoside. Bacteria were allowed to express the recombinant protein overnight at room temperature, followed by harvesting by centrifugation at 6000rpm for 10 min. The pellet was resuspended in 10 ml of resuspension buffer (20 mM NaHPO4, 300mM NaCl, 20mM Imidazole, pH 7.8), and cell lysis was induced by freezing at -80°C with PMSF, Leupeptin and Pepstatin added to inhibit protease activity. The mixture was sonicated on ice with 0.1% Triton X-100 for 30 min, followed by centrifugation at 13,000rpm for 30 min. The supernatant was mixed with 300µl of Ni-NTA agarose (Qiagen), and the proteins were allowed to bind for 3 hours, followed by washing with 20 ml of resuspension buffer, and eluted with 500 µl of elution buffer (20 mM NaHPO4, 300mM NaCl, 10% glycerol, 150mM Imidazole, pH7.8).
Protein concentrations were determined via absorption at 280 nm and with the QuickStart Bradford Protein Assay kit (Bio-Rad) after protein denaturation with 2M Guanidine HCI, using the NanoDrop 1000 spectrophotometer (ThermoFisher Scientific). The absorption spectra were measured on the Cary 3e (Varian). Fluorescence emission spectra were measured on the Cary Eclipse (Varian), with quantum efficiency determined using purified mCherry protein as a reference (purified as above).

### mRNA synthesis, injection and zebrafish image recording

All open reading frames of fluorescent proteins were subcloned into the pCS2+ expression vector (Rupp et al, 1994). Notl-templates were generated for mRNA transcription using the SP6 mMessageMachine Kit (Ambion). After purification with RNeasy (Qiagen) columns, mRNA was injected at either 100 ng/µl or 250 ng/µl into one-cell stage embryos, which were raised in 30% Danieua/0.15mM phenylthiourea (PTU). At 24 hours post fertilization (hpf), fluorescent embryos were dechorionated, mounted on coverslips in 1.2% ultra-low gelling agarose, overlaid with 30% Danieua/PTU and analysed by laser scanning confocal microscopy (Zeiss LSM510 Meta).

### Deep tissue imaging

The samples were excited with a NdYAG-pumped wavelength-tunable dye laser (Sirah Laser, Kaarst, Germany) and imaged with a f#1.2 50mm lens (Nikkor, Japan) and a low-noise cooled 512x512 16-bit CCD camera (VersArray, Princeton Instruments, Acton MA), using 10 nm FWHM bandpass interference filters (Chroma, Bellows Falls, VT) centered at 610, 670 and 710 nm. Imaging was performed in a dark room. Approximately 1 million cells expressing Amrose variants and mRaspberry, as well as control cells, were inserted in the bottom of a small capillary tube with 0.9 mm diameter and centrifuged to occupy approximately 3 uL of volume. First, the tubes were imaged in reflectance mode against a dark background for a series of excitation wavelengths and emission filters to establish a baseline of fluorescent protein expression. Following, the tubes were inserted in the esophagus of a euthanized mouse with the cells placed above the heart. The mouse tissue was illuminated at the dorsal side and the emerging photons were imaged from the ventral side in transillumination mode. The total tissue thickness from illumination point to chest was approximately 1.5 cm with the esophagus being approximately in the mid-distance. For all images acquired the camera dark noise was subtracted and the pixel counts were normalized for exposure time (5 sec), laser intensity (20-200 mW), and transmission of interference filters. The auto-fluorescence images were subtracted from fluorescence to get the net fluorescence. The fluorescent images were overlaid on background white-light images using a variable transparency function. The intensity of the fluorescent proteins was measured by summing up the pixel values in a selected region of interest in the area on the maximum signal.

### Example 2: In vivo activity of the Amrose variants in zebrafish

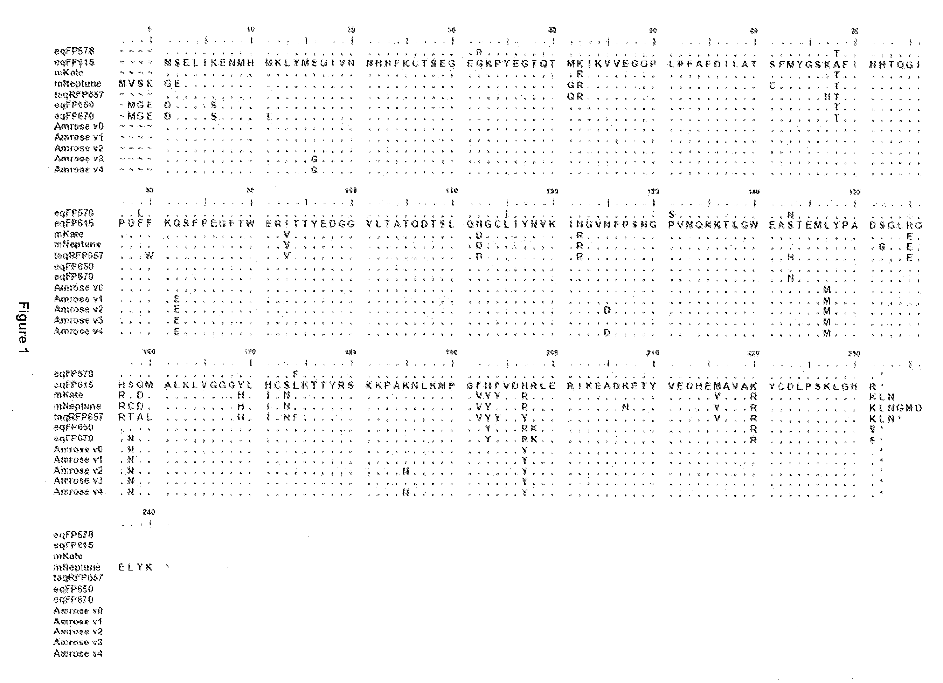

Following initial sequencing and testing of the excitation and emission spectrum of the original candidates, three related clones harboring variations (identified as v1, v2 and v3) were selected to further test for applicable use. Joining these three variants were an engineered back mutation (E82Q) denoted v0 and a forward mutation (adding v3's E16G to v2) denoted v4 (Figure 1).

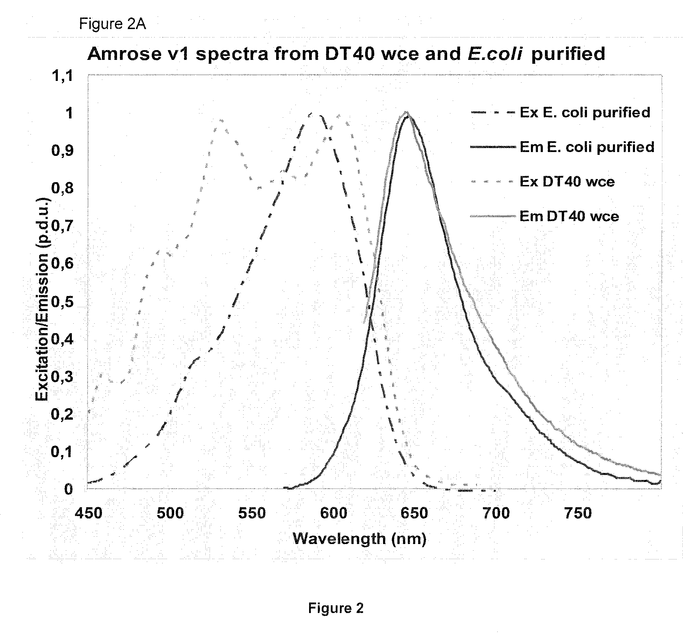

Amrose v0-v4 variants were cloned into the pRSETa (Invitrogen) vector system to isolate pure Amrose variant fluorescent protein for analysis as well as whole cell extracts of v1 from DT40 cells (Figure 2A, 2B and 2C). The data shows that bacterially expressed Amrose protein differs from eukaryotic expression. Amrose v1 expressed in whole cell extracts of DT40 have Ex/Em peaks of 606 nm/645 nm, whereas expression from bacteria suggests Ex/Em peaks of 589 nm/645 nm. In addition, not only are the absorption peaks shifted lower, but v0 and v4 do in fact emit and v4 emits strongly when expressed from a bacterial system.

To demonstrate Amrose functionality in an established higher model system, Amrose v0-v3 variants were cloned into the pCS2+ vector system to synthesize mRNA by *in vitro* transcription which was used for injection into one-cell stage zebrafish embryos. For direct comparison, equal amounts of Amrose variants 0-3, HcRed, mPlum and mCherry mRNA were used for injection. Image recording was performed with an excitation wavelength of 633 nm to scan the respective emission profiles and recording the emission in 10 nm windows from 660 to 780 nm. This revealed that Amrose variants 1-3 showed a brighter emission above 650 nm and thus in the preferred optical window for deep tissue imaging than v0 or any of the three commercially available fluorescent proteins (Figure 3A and 3B). Amrose v3, being the brightest in the zebrafish embryo, was further analyzed. Photostability analysis was performed using three zebrafish embryos each for Amrose v3 and mCherry. The embryos were excited using a 633 nm laser set at 100% power and fast scanned producing a series of 150 pictures. The first and last picture of each set measured had ten single corresponding spots compared for loss of emission intensity (Figure 3C). Consistent with spectroscopy measurements, Amrose v3 can be efficiently excited with the commonly used yellow (561, 594 nm) or red laser (633 nm) excitation wavelengths as demonstrated here by image recording of the embryonic retina or skeletal muscle in the trunk (Figure 4).

### Example 3: In vivo activity of the Amrose mimicking live mouse imaging

For the optical tomography and deep tissue imaging experiments, approximately one million DT40 cells expressing either eqFP615 or Amrose v1 (and non-expressing cells for control) were inserted into a 0.9 mm capillary tube and centrifuged to form a small pellet. The capillary tube was inserted into the esophagus of a euthanized mouse and imaged in transillumination mode mimicking the procedure designed for live mouse imaging in the near-infrared (Deliolanis et al., 2009). In the experiment (Figure 5A), a 625 nm laser beam illuminated the upper mouse torso just below the neck and the fluorescence emitted from the opposite side is imaged using a 635/670 nm filter set, a lens and a CCD camera. The capillary tube with the Amrose expressing cells was removed and replaced with a similar tube containing eqFP615 expressing cells for comparison and the non-expressing cells for control. In order to eliminate the tissue autofluorescence, the fluorescence image from the control cells is subtracted from the corresponding fluorescence image to yield the net fluorescence.
For quantifying the performance of Amrose in deep tissue optical imaging experiments, DT40 cells expressing no FP (control), Amrose v1 and mRaspberry were used. Both Amrose and mRaspberry were expressed at comparable levels under the direction of the RSV promoter and in a non-mutating context. Approximately one million cells of each clone were inserted into 0.9 mm capillary tubes and centrifuged to form a small pellet; this amount corresponds to a small primary tumor, or average size metastasis. The capillary tube was inserted into the esophagus of a euthanized mouse and imaged in transillumination mode mimicking the procedure designed for live mouse imaging in the near-infrared (Deliolanis *et al,* 2008). The capillary tube with the Amrose v1 expressing DT40 cells was removed and replaced with a similar tube containing non-fluorescing DT40 cells as control and mRaspberry for comparison. In order to eliminate the tissue auto-fluorescence, the background fluorescent image from the control cells is subtracted from the corresponding Amrose v1 and mRaspberry fluorescent images to yield the net fluorescence of the FPs. The fluorescent images (Figure 5B) from the FPs for 2 different excitation and emission wavelengths corresponding to available laser diode wavelengths are presented. At 590 nm, mRaspberry is excited close at its peak wavelength, still the detected signal is marginally above the noise level and Amrose signal is below noise level and practically undetectable. At 635 nm, there is a one order of magnitude increase for the mRaspberry signal and Amrose signal is even 2.25 times brighter than mRaspberry.

A similar experiment was also performed to test the feasibility of imaging Amrose in a Fluorescence Molecular Tomography (FMT) system (Deliolanis *et al,* 2009) that allows the tomographic reconstruction of fluorescence biodistribution (Figure 6). The details are as previous except that the DT40 cells used were expressing Amrose v2 and there was no comparison FP for this wavelength. The tissue was excited by a 670 nm continuous wave laser (B&WTek, Newark, DE) and the emanating photon field is imaged at 710 nm. This clearly shows that Amrose v2 can be excited at 670 nm and the emitted fluorescence can be detected through mouse tissue.

### Further References

Arakawa H, Kudo H, Batrak V, Caldwell RB, Rieger MA, Ellwart JW, Buerstedde JM. (2008 ) Protein evolution by hypermutation and selection in the B cell line DT40. Nucleic Acids Res. 36(1):e1.
Bebbington CR, Renner G, Thomson S, King D, Abrams D, Yarranton GT. (1992) High-level expression of a recombinant antibody from myeloma cells using a glutamine synthetase gene as an amplifiable selectable marker. Biotechnology 10(2):169-175.
Chalfie, M., Tu, Y., Euskirchen, G., Ward, W.W., and Prasher, D.C. (1994) Green fluorescent protein as a marker for gene expression, Science, 263, 802-805
Ckurshumova W, Caragea AE, Goldstein RS, Berleth T. (2011) Glow in the dark: fluorescent proteins as cell and tissue-specific markers in plants. Mol Plant. 4(5):794-804. Review.
Davenport, D. and Nicol, J. A. C. (1955) Luminescence in Hydromedusae, Proc. R. Soc. Lond. B November 29, 1955 144:399-411
Deliolanis NC, Kasmieh R, Wurdinger T, Tannous BA, Shah K, Ntziachristos V. (2008) Performance of the red-shifted fluorescent proteins in deep-tissue molecular imaging applications. J Biomed Opt. 13(4):044008.
Deliolanis NC, Dunham J, Wurdinger T, Figueiredo JL, Tannous BA, Ntziachristos V. (2009) In-vivo imaging of murine tumors using complete-angle projection fluorescence molecular tomography. J Biomed Opt 14(3):030509.
Doetschman TC, Eistetter H, Katz M, Schmidt W, Kemler R. (1985 ) The in vitro development of blastocyst-derived embryonic stem cell lines: formation of visceral yolk sac, blood islands and myocardium. J Embryol Exp Morphol. 87:27-45.
Hooper M, Hardy K, Handyside A, Hunter S, Monk M. (1987) HPRT-deficient (Lesch-Nyhan) mouse embryos derived from germline colonization by cultured cells. Nature 326(6110):292-5.
Inouye, S. and Tsuji, F.I. (1994) Aequorea green fluorescent protein. Expression of the gene and fluorescence characteristics of the recombinant protein, FEBS Lett., 341, 277-280.
Lin MZ, McKeown MR, Ng HL, Aguilera TA, Shaner NC, Campbell RE, Adams SR, Gross LA, Ma W, Alber T, Tsien RY.( 2009) Autofluorescent proteins with excitation in the optical window for intravital imaging in mammals. Chem Biol. 16(11):1169-79.
Morozova KS, Piatkevich KD, Gould TJ, Zhang J, Bewersdorf J, Verkhusha VV. (2010) Far-red fluorescent protein excitable with red lasers for flow cytometry and superresolution STED nanoscopy. Biophys J 99(2):L13-5.
Murphy G, Houbrechts A, Cockett MI, Williamson RA, O'Shea M, Docherty AJP. (1991) The N-terminal domain of tissue inhibitor of metalloproteinases retains metalloproteinase inhibitory activity. Biochemistry 30, 8097-8102.
Ntziachristos V, Razansky D. (2010) Molecular imaging by means of multispectral optoacoustic tomography (MSOT). Chem Rev. 110(5):2783-94.
Parrish JA. (1981) New concepts in therapeutic photomedicine: photochemistry, optical targeting and the therapeutic window. J Invest Dermatol. 77(1):45-50.
Pletnev S, Shcherbo D, Chudakov DM, Pletneva N, Merzlyak EM, Wlodawer A, Dauter Z, Pletnev V. (2004) A crystallographic study of bright far-red fluorescent protein mKate reveals pH-induced cis-trans isomerization of the chromophore. J Biol Chem 283(43):28980-7
Prasher, D.C., Eckenrode, V.K., Ward, W.W., Prendergast, F.G., and Cormier, M.J. (1992) Primary structure of the Aequorea victoria green-fluorescent protein, Gene, 111, 229-233.
Razansky D, Buehler A, Ntziachristos V. (2011) Volumetric real-time multispectral optoacoustic tomography of biomarkers. Nat Protoc. 6(8):1121-9
Robertson E, Bradley A, Kuehn M, Evans M. (1986) Germ-line transmission of genes introduced into cultured pluripotential cells by retroviral vector. Nature 323(6087):445-8.
Shcherbo D, Merzlyak EM, Chepurnykh TV, Fradkov AF, Ermakova GV, Solovieva EA, Lukyanov KA, Bogdanova EA, Zaraisky AG, Lukyanov S, Chudakov DM (2007) Bright far-red fluorescent protein for whole-body imaging. Nat Methods. 4(9):741-6.
Shcherbo D, Murphy CS, Ermakova GV, Solovieva EA, Chepurnykh TV, Shcheglov AS, Verkhusha VV, Pletnev VZ, Hazelwood KL, Roche PM, Lukyanov S, Zaraisky AG, Davidson MW, Chudakov DM. (2009) Far-red fluorescent tags for protein imaging in living tissues. Biochem J. 418(3):567-74.
Shcherbo D, Shemiakina II, Ryabova AV, Luker KE, Schmidt BT, Souslova EA, Gorodnicheva TV, Strukova L, Shidlovskiy KM, Britanova OV, Zaraisky AG, Lukyanov KA, Loschenov VB, Luker GD, Chudakov DM. (2010) Near-infrared fluorescent proteins. Nat Methods 7(10):827-9.
Shimomura, O., Johnson, F.H., and Saiga, Y. (1962) Extraction, Purification and Properties of Aequorin, a Bioluminescent Protein from Luminous Hydromedusan, Aequorea, J. Cell. Comp. Physiol., 59, 223-239.
Shu X, Royant A, Lin MZ, Aguilera TA, Lev-Ram V, Steinbach PA, Tsien RY. (2009) Mammalian expression of infrared fluorescent proteins engineered from a bacterial phytochrome. Science. 324(5928):804-7.
Soriano P. (1997) The PDGFα receptor is required for neural crest cell development and for normal patterning of the somites. Development 124:2691-2700.

## Claims

1. A nucleic acid molecule encoding a polypeptide having a fluorescence emission activity at a wavelength of at least 630 nm, wherein said nucleic acid molecule is selected from the group consisting of
(a) a nucleic acid molecule encoding a polypeptide having the amino acid sequence of SEQ ID NO: 1;
(b) a nucleic acid molecule encoding a polypeptide having the amino acid sequence of SEQ ID NO: 1, with the exception that in the polypeptide of SEQ ID NO: 1
(b-i) glutamic acid at the position corresponding to position 16 is replaced by glycine;
(b-ii) asparagine at the position corresponding to position 125 is replaced by aspartic acid; and/or
(b-iii) lysine at the position corresponding to position 185 is replaced by asparagine;
(c) a nucleic acid molecule encoding a polypeptide having at least 94% sequence identity to the polypeptide encoded by the nucleic acid molecule of (a) or (b);
(d) a nucleic acid molecule having the nucleotide sequence of SEQ ID NO: 2;
(e) a nucleic acid molecule having the nucleotide sequence of SEQ ID NO: 2, with the exception that said nucleic acid molecule has
(e-i) at the positions corresponding to positions 48 to 50 of SEQ ID NO: 2 a nucleotide triplet selected
from the group consisting of GGT, GGC, GGA and GGG;
(e-ii) at the positions corresponding to positions 375 to 377 of SEQ ID NO: 2 a nucleotide triplet selected from the group consisting of GAT and GAC; and/or
(e-iii) at the positions corresponding to positions 555 to 557 of SEQ ID NO: 2 a nucleotide triplet selected from the group consisting of AAT and AAC;
(f) a nucleic acid molecule having the nucleotide sequence of (d) or (e), wherein each thymine is replaced by uracile; and
(g) a nucleic acid molecule degenerate with respect to the nucleic acid molecule of (d), (e) or (f).

2. A vector comprising the nucleic acid molecule of claim 1.

3. A non-human host transformed with the vector of claim 2.

4. A method of producing a polypeptide having a fluorescence emission activity at a wavelength of at least 630 nm, the method comprising culturing the host of claim 3 under suitable conditions and isolating the polypeptide produced.

5. A polypeptide encoded by the nucleic acid molecule of claim 1 or produced by the method of claim 4.

6. A fusion protein comprising the polypeptide of claim 5.

7. The fusion protein of claim 6, wherein the polypeptide of claim 7 is fused to a His-tag, a Strep-tag, a GST-tag, a TAP tag, biotin, an HA tag or a signal sequence for intra- or extracellular targeting.

8. A diagnostic composition comprising at least one of
(a) the nucleic acid molecule of claim 1;
(b) the vector of claim 2;
(c) the polypeptide of claim 5; or
(d) the fusion protein of claim 6 or 7.

9. A method of detecting the expression of a gene of interest in a sample, the method comprising providing a light stimulus to the sample and detecting the fluorescence emitted by a polypeptide encoded by the nucleic acid molecule of claim 1, wherein said nucleic acid molecule is
(a) operably linked to a promoter controlling said gene of interest; or
(b) fused to said gene of interest.

10. A method of detecting the activity of a promoter of interest in a sample, the method comprising providing a light stimulus to the sample and detecting the fluorescence emitted by the polypeptide encoded by the nucleic acid molecule of claim 1, wherein said nucleic acid molecule is operably linked with said promoter of interest.

11. A method of detecting the presence of a protein of interest, the method comprising contacting a sample with the fusion protein of claim 6, wherein said fusion protein comprises a polypeptide specifically binding to the protein of interest, providing a light stimulus to the sample and detecting the fluorescence emitted by the fusion protein.

12. A method of detecting the localization of a polypeptide of claim 5 or a fusion protein according to claim 6 or 7 in a cell or tissue, the method comprising providing said cell or tissue with a light stimulus and detecting the location of the polypeptide or fusion protein by
(a) detecting the fluorescence emitted by the polypeptide or fusion protein upon excitation by the light stimulus; and/or
(b) detecting the temperature change produced by the polypeptide or fusion protein upon absorption of the light stimulus.

13. A kit comprising at least one of
(a) the nucleic acid molecule of claim 1;
(b) the vector of claim 2;
(c) the host of claim 3;
(d) the polypeptide of claim 5; or
(e) the fusion protein of claim 6 or 7.
